# FASCICULE DE BREVET EUROPEEN

(11) **EP 2 661 213 B1**
(45) Date de publication et mention de la délivrance du brevet: **15.06.2016**
(21) Numéro de dépôt: 11813429.5
(22) Date de dépôt: 23.12.2011
(51) Int. Cl.: A61B 5/00, A61B 10/00

(54) **SYSTÈME ET PROCÉDÉ POUR DÉTECTER LA SENSIBILITÉ D'UNE PERSONNE A UNE OU PLUSIEURS SUBSTANCES POSSIBLEMENT ALLERGISANTES**
SYSTEM UND VERFAHREN ZUR ERKENNUNG DER EMPFINDLICHKEIT EINER PERSON GEGENÜBER EINER ODER MEHREREN POTENZIELL ALLERGENEN SUBSTANZEN
SYSTEM AND METHOD FOR DETECTING THE SENSITIVITY OF A PERSON TO ONE OR MORE POTENTIALLY ALLERGENIC SUBSTANCES

(30) Priorité: 04.01.2011 FR 1100026; 20.05.2011 FR 1101549; 30.06.2011 US 201113173805
(43) Date de publication de la demande: 13.11.2013
(73) Titulaire: Ergylink Sarl, 92300 Levallois-perret (FR)
(72) Inventeur: GILBERT, Jerome, F-92300 Levallois-perret (FR)
(86) Numéro de dépôt international: PCT/IB2011/055930
(87) Numéro de publication internationale: WO 2012/093315

(56) Documents cités:
- WO-A1-01/24994
- WO-A2-2007/008824

## Description

### Domaine technique

L'invention se situe dans le domaine de l'aide au diagnostic des allergies.

### Etat de la technique antérieure

On connaît des solutions pour détecter la sensibilité d'une personne à une ou plusieurs substances possiblement allergisantes qui sont longues et fastidieuses. Le document WO2007/008824 décrit un système de test d'allergie et un procédé pour l'exploiter. Le document WO01/24994 décrit un procédé de marquage des lentilles optiques moulées pour faciliter notamment les contrôles d'inventaire dans des stocks.

### Exposé de l'invention

La présente invention, qui est définie dans les revendications, se situe dans le domaine de l'analyse des caractéristiques de la peau. La peau au sens de l'invention comprenant l'ensemble des constituants, des annexes et des fonctionnalités de l'organe. Il est ainsi prévu par exemple que l'invention soit utilisée pour l'analyse des cheveux. Des applications de l'invention sont prévues pour la prescription de cosmétiques et/ou d'actifs et/ou de soins en fonction d'au moins une caractéristique de la peau. Des applications de l'invention sont aussi prévues pour le dépistage du cancer de la peau. Il est en effet prévu d'utiliser l'invention dans le cadre de la détection du mélanome. Les grains de beauté peuvent être ainsi surveillés sur la base des actions suivantes : acquisition périodique d'une image numérique et mémorisation des informations correspondantes, comparaison automatique de l'évolution entre les captures d'image, calcul de la vitesse d'évolution et génération d'alerte(s) invitant à la consultation de médecins spécialisés, prise de rendez-vous automatique possible avec exploitation de la géolocalisation et du degré d'urgence lié à l'importance du problème détecté. En effet, un mélanome peut évoluer très rapidement et mettre en jeu la vie de la personne. La fréquence usuelle des visites de contrôle chez un dermatologue est généralement trop faible au regard du risque encouru. L'invention permet aux personnes à risque, de se surveiller autant de fois que nécessaire sans la contrainte de la prise d'un rendez-vous et de la difficulté de trouver une date rapprochée et ceci à moindre coût pour les systèmes de financement de la santé.

Par ailleurs, la progression du parc de téléphones portables est phénoménale dans le mode entier avec un taux d'équipement moyen des personnes, tous pays confondus, qui est actuellement globalement de l'ordre de 60% et qui est proche de 80% dans la plupart des pays développés. La plupart des modèles récents de téléphones portables embarquent une fonction d'appareil photo numérique. En outre, le segment des Smartphones aptes à exécuter des applications spécifiques, tiré par l'emblématique série des iPhone d'Apple (marques déposées), par les appareils compatibles avec le système Android de Google (marques déposées) ou encore par les Blackberry de Research In Motion (marques déposées), est en croissance forte.

D'autres types d'appareils électroniques équipés d'une fonction d'acquisition d'image sont à la disposition du grand public et, utilisés seuls ou avec des accessoires ou encore en combinaison, sont aptes à mettre en oeuvre l'invention. Il s'agit par exemple des tablettes numériques telles que l'iPad d'Apple (marques déposées), de baladeurs tels que certains iPod d'Apple (marques déposées), des ordinateurs portables avec caméra incorporée ou ajoutée, des ordinateurs fixes avec webcam ajoutée, des appareils photos et caméscopes numériques, des téléviseurs connectés avec caméra incorporée ou ajoutée etc.

L'invention prévoit un système pour analyser au moins une caractéristique de la peau par des moyens relevant de l'optique et du traitement d'image.

Le système selon l'invention comprend :
- un appareil électronique grand public, apte à acquérir au moins une image par des moyens numériques et apte à produire des données en relation avec l'au moins une image acquise ; et
- des moyens pour traiter lesdites données en relation avec l'au moins une image acquise dans le but de produire une information en rapport avec au moins une caractéristique de la peau.

L'invention prévoit que le système comprend en outre :
- des moyens pour produire un grossissement optique de l'image.

Dans le cadre d'une première variante de mise en oeuvre, l'invention prévoit que les moyens pour produire un grossissement optique de l'image comprennent :
- des moyens pour rendre l'image nette lors d'une acquisition d'image à une distance plus courte que la plus courte distance possible pour laquelle ledit appareil électronique grand public a été conçu; et
- des moyens pour fixer temporairement lesdits moyens pour rendre l'image nette sur ledit appareil électronique grand public de sorte que les moyens pour rendre l'image nette soient placés de manière appropriée relativement à l'objectif de l'appareil électronique grand public.

Dans le cadre d'une seconde variante de mise en oeuvre, l'invention prévoit que les moyens pour produire un grossissement optique de l'image comprennent :
- des moyens pour rendre l'image nette indépendamment de la distance à laquelle se trouve l'objectif de l'appareil électronique grand public utilisé pour acquérir l'image; et
- des moyens pour maintenir temporairement les moyens pour rendre l'image nette en position fonctionnelle sur la peau.

On entend par analyse d'au moins une caractéristique de la peau, tout procédé permettant de produire des informations quantitative ou qualitative en relation avec au moins une caractéristique de la peau. Il est à noter cependant que l'invention prévoit par la mise en oeuvre de moyens tels que des bases de données permettant de lier des caractéristiques entre elles, de produire des informations sur une caractéristique d'intérêt de la peau qui n'est pas celle sur laquelle l'analyse proprement dite a porté.

On entend par caractéristique de la peau toute caractéristique physique, chimique ou biologique de la peau dont l'analyse présente une utilité dans le domaine d'application de l'invention. La peau étant entendue dans sa définition la plus large incluant l'ensemble des tissus et des constituants de cet organe dont les phanères tels que les ongles, les poils et les cheveux ainsi que l'écosystème de la peau, tout produit en relation avec la peau et toute manifestation pathologique de la peau.

On entend par traitement d'image tout traitement des données résultant de l'acquisition de l'image par des moyens numériques. Par exemple tout algorithme appliqués aux pixels de l'image extraits du fichier résultant de l'acquisition d'une photo numérique. Il est aussi prévu dans l'invention d'appliquer des traitements aux trames d'un flux vidéo stocké sous la forme d'un fichier ou en cours de capture.

On entend par appareil électronique grand public, apte à acquérir au moins une image par des moyens numériques et apte à produire des données en relation avec l'au moins une image acquise, tout appareil accessible à la population générale et qui comprend des moyens permettant de capturer des images numériques une par une, en séquence ou sous la forme d'un flux continu. En d'autres termes, il s'agit de tout appareil qui n'a pas été conçu spécifiquement pour l'analyse de la peau à destination des professionnels. Bien entendu, il est prévu que les solutions de l'invention puissent être aussi utilisées par des professionnels. Il s'agit par exemple d'appareils électroniques comprenant une fonction appareil photo ou caméra vidéo numérique. Les appareils électroniques grand public utilisables dans le cadre de l'invention sont par exemple des téléphones cellulaires simples, des Smartphones, des tablettes numériques, des baladeurs, des microordinateurs ou des téléviseurs équipés d'une fonction appareil photo ou caméra numérique. On ne sort cependant pas de l'invention dans le cas de l'assemblage d'une pluralité d'appareils électroniques grand public pour mettre en oeuvre les fonctions nécessaires à l'invention. Par exemple il est prévu de pouvoir utiliser une webcam connectée à un microordinateur ou à une tablette ou encore un appareil photo ou un caméscope numérique pour acquérir les images dans le cadre d'une première étape d'acquisition sans traitement d'image que complète l'utilisation d'une tablette numérique ou d'un microordinateur pour prendre en charge une étape de traitement d'image local et/ou une étape de transmission d'informations vers un serveur par l'intermédiaire d'un réseau pour exécuter des traitements d'image distants.

Dans le cadre de la dite première variante de mise en oeuvre, on entend par moyens pour rendre l'image nette lors d'une acquisition d'image à une distance plus courte que la plus courte distance possible pour laquelle ledit appareil électronique grand public a été conçu, tout dispositif optique interposé entre l'objectif de l'appareil électronique et l'objet de l'acquisition d'image, tous moyens mis en oeuvre sous la forme d'au moins un logiciel, et toute combinaison d'au moins un dispositif optique et d'au moins un logiciel. Par convention dans la suite de ce document, on appellera dispositif selon l'invention ou dispositif pour rendre l'image nette, toute mise en oeuvre sous une forme matérielle des moyens pour rendre l'image nette lors d'une acquisition d'image à une distance plus courte que la plus courte distance possible pour laquelle ledit appareil électronique grand public a été conçu. Il peut s'agir par exemple d'une simple lentille convergente placée devant l'objectif ou d'un groupe de lentilles. Il est prévu dans l'invention que les lentilles mises en oeuvre soient réalisées en verre ou en matière plastique de qualité optique moulée pour réduire les coûts. Dans certaines variantes de mise en oeuvre plus particulièrement destinées à la cosmétique, il est prévu une optique permettant de rendre l'image nette avec une relativement grande profondeur de champ pour permettre des prises de vues nettes de surfaces de peau d'intérêt qui ne sont pas planes telles que par exemple le contour de l'oeil, les paupières, la commissure des lèvres, le front, le cou. Il est prévu que les moyens pour rendre l'image nette lors d'une acquisition d'image à une distance plus courte que la plus courte distance possible pour laquelle ledit appareil électronique grand public a été conçu soient mis en oeuvre sous la forme d'au moins un logiciel par exemple dans le cas d'appareils électroniques grand public dont la distance minimale de mise au point automatique est bloquée à une distance trop grande par rapport aux besoins de l'invention. Il arrive que les constructeurs d'appareils électroniques bloquent la capacité à prendre des photos de leurs appareils avant l'apparition de distorsions visibles dans l'image alors que ces appareils pourraient techniquement acquérir des images à des distances plus courtes. Dans des applications non artistiques comme celles de l'invention, les distorsions apparaissant à courte distance ne sont pas gênantes ou peuvent être corrigées par traitement d'image. Dans de tels cas, selon le type d'appareil électronique grand public, un logiciel d'application spécifique ou une nouvelle version de logiciel enfoui (firmware en langue anglaise) permet de débloquer les limitations de distance mises en oeuvre par les constructeurs des appareils. Il est à noter que de telles limitations de la distance d'acquisition d'image dans les appareils grand public ne sont généralement mises en oeuvre qu'en mode photographie et que dans le mode vidéo souvent proposé, cette limitation n'est pas mise en oeuvre. Cela étant, le mode photographie sera préféré lorsque cela sera possible car il permet en général d'acquérir des images ayant une plus grande résolution que le mode vidéo associé.

On entend par moyens pour fixer temporairement lesdits moyens pour rendre l'image nette sur ledit appareil électronique grand public, tout moyen de fixation permettant une adaptation à la conformation dudit appareil électronique de sorte à aligner les axes optiques des moyens pour rendre l'image nette et de l'objectif de l'appareil électronique. Les moyens de fixation sont en outre prévus pour respecter l'intégrité de l'appareil électronique. Il est prévu par exemple d'utiliser une pellicule adhésive dite repositionnable c'est-à-dire qui colle à la surface de l'appareil mais que l'application d'une faible force permet de décoller sans abîmer la surface de l'appareil. De manière avantageuse, l'application d'un primaire d'adhésion approprié sur la surface correspondante des moyens pour rendre l'image nette puis la pose d'un film de protection pelable sur la surface externe de la pellicule adhésive repositionnable pendant la fabrication permet d'obtenir à la fois une adhésion permanente de la pellicule sur les moyens pour rendre l'image nette et une adhésion de type repositionnable sur la surface destinée au contact avec la surface externe de l'appareil électronique. Il est également prévu des moyens pour fixer temporairement lesdits moyens pour rendre l'image nette sur ledit appareil électronique grand public, qui n'utilisent pas d'adhésif mais moyens entièrement mécaniques. Il est prévu dans certaine variantes que les moyens mécaniques soient réglables pour adapter lesdits moyens pour rendre l'image nette à une pluralité de modèles d'appareils électroniques grand public. De tels moyens mécaniques utilisent des découpes appropriées dans des éléments des moyens pour rendre l'image nette, ces moyens étant alors maintenus solidaire de l'appareil électronique par les forces de friction résultant d'un montage séré. Il est également prévu d'utiliser au moins un bracelet en matière élastique dans certaines variantes particulièrement économiques. Dans d'autres variantes, il est prévu que les lesdits moyens pour rendre l'image nette soient mécaniquement conçus pour s'adapter parfaitement à un seul modèle ou à une famille d'appareils électroniques grand public particulièrement répandu, par exemple une conception dédiée à un ou plusieurs modèles « d'iPhone » ou à un ou plusieurs modèles « d'iPod » (marques déposées d'Apple Computer) équipés d'une camera ou encore à un ou plusieurs modèles de « Blackberry » (marque déposée de Research In Motion).

Dans le cadre de ladite seconde variante de mise en oeuvre, on entend par moyens pour rendre l'image nette indépendamment de la distance à laquelle se trouve l'objectif de l'appareil électronique grand public utilisé pour acquérir l'image, des moyens optiques qui font apparaitre grossie et nette l'image de la surface de peau et des éléments annexes selon l'invention qui sont inclus dans l'image le cas échéant. Les moyens optiques sont agencés pour que la netteté de l'image fournie par le dispositif ne change pas en fonction de la distance à laquelle se trouve l'objectif de l'appareil électronique. Seul change le rapport des surfaces de l'image d'intérêt visible sur la face externe principale du dispositif selon l'invention par rapport à la surface restante de l'image acquise lorsqu'on modifie la distance entre le dispositif et l'appareil électronique. Cette seconde variante suppose des composants optiques d'une relativement grande surface pour que la taille de l'image visible par l'appareil électronique dans l'image d'ensemble à acquérir comprenne suffisamment de pixels pour la rendre apte aux traitements d'image selon l'invention. En pratique, une image de l'ordre de trois centimètres sur cinq convient pour une acquisition par la plupart des appareils électroniques. Les lentilles de Fresnel sont les composants optiques préférés pour mettre en oeuvre cette variante de l'invention en ce qu'ils permettent de réaliser des lentilles de grande surface à la fois performantes dans le contexte de l'invention et de faible épaisseur. Les lentilles de Fresnel offrent les avantages supplémentaires d'être flexibles et de faible coût lorsqu'elles sont réalisées dans des feuilles de matière plastique de qualité optique. Cette seconde variante de mise en oeuvre du dispositif selon l'invention offre en outre l'avantage de fournir une image visible à l'oeil nu sans nécessiter le recours aux moyens de monitoring d'un appareil électronique. Dans le cadre de l'application à la détection du cancer de la peau, cette variante peut dans certaines conditions dispenser l'utilisateur d'acquérir une ou plusieurs images d'ensemble d'une plus large partie du corps pour localiser les grains de beauté ou les lésions suivies. Ceci parce que l'image issue des moyens de l'invention est naturellement comprise dans une image d'une plus grande surface de peau qui permet en général de localiser l'endroit du corps où elle se situe.

On entend par moyens pour maintenir temporairement les moyens pour rendre l'image nette en position fonctionnelle sur la peau, tout moyen qui permet de libérer les mains de l'utilisateur pour maintenir le dispositif dans une position apte à l'acquisition d'image. L'utilisateur pouvant ainsi avantageusement utiliser ses deux mains pour la manipulation de l'appareil électronique aux fins d'acquérir l'image. Par exemple, il est prévu des moyens consommables comprenant un film adhésif repositionnable des deux côtés. L'adhésif étant avantageusement compatible avec une application sur la peau. Cette solution technique et particulièrement préféré dans le cadre de moyens légers et à faible coût utilisant des lentilles de Fresnel réalisées dans des feuilles en matière plastique. Le dispositif ainsi constitué pouvant tenir ainsi sans risque de chute sur une surface de peau verticale. On ne sort cependant pas du cadre de l'invention lorsqu'on utilise l'effet de la pesanteur pour maintenir le dispositif en position. Le dispositif est alors simplement posé sur la surface de peau d'intérêt. A charge à la personne d'adopter la position appropriée compte tenu de la localisation de la surface de peau d'intérêt pour que le dispositif se maintienne en position sous l'effet de la pesanteur.

Le système selon l'invention prévoit en outre que lesdits moyens pour rendre l'image nette comprennent des moyens pour offrir un grossissement optique élevé de l'image. L'interposition des moyens pour rendre l'image nette lors d'une acquisition d'image à une distance plus courte que la plus courte distance possible pour laquelle ledit appareil électronique grand public a été conçu, induit naturellement un premier niveau de grossissement optique par rapport aux caractéristiques nominales de l'appareil par la modification des rapports de distance. En outre, la résolution des caméras embarquées dans les appareils électronique grand public augmente à chaque génération d'appareils à surface de capteur égale voir à surface de capteur plus réduite. Ceci fait que le grossissement naturel obtenu grâce à l'interposition des moyens pour rendre l'image nette est généralement suffisant pour appliquer les traitements d'image selon l'invention et en obtenir les résultats attendus. Dans certain cas cependant il est prévu d'accroitre le grossissement naturel apporté par les moyens pour rendre l'image nette par l'ajout de moyens optiques supplémentaires permettant un grossissement élevé associé à un niveau d'aberration optique compatible avec les traitements d'image selon l'invention. Par exemple un grossissement optique élevé est nécessaire pour l'analyse selon l'invention des cheveux.

Le système selon l'invention prévoit que les moyens pour produire un grossissement optique de l'image comprennent en outre des moyens pour transformer l'expression d'au moins une caractéristique physique, chimique ou biologique de la peau de sorte à permettre une appréciation de l'au moins une caractéristique physique, chimique ou biologique de la peau par traitement d'image. Cette caractéristique technique est particulièrement utile dans le cas des applications de l'invention dans le domaine de la cosmétique. L'invention prévoit par exemple d'utiliser des réactifs chimiques qui changent de couleur selon une caractéristique chimique comme l'acidité, la quantité de sébum ou encore une caractéristique physique comme le degré d'humidité en surface. Il est également prévu des réactifs aptes à changer de couleur en fonction de caractéristiques d'ordre biologique telles qu'en rapport avec le type de microorganismes colonisant la surface de peau analysée dont la connaissance peut être exploitée dans le domaine de la cosmétique. Lesdits réactifs étant placés sur un support qui est à la fois au contact de la peau à analyser et dans le plan de l'image acquise par l'appareil électronique. De manière particulièrement avantageuse, il est prévu que le support desdits réactifs soit solidaire desdits moyens pour rendre l'image nette. Il est aussi prévu sur le support le marquage d'un code apparaissant dans le plan de l'image acquise par l'appareil électronique pour permettre aux algorithmes de traitements d'image de déterminer le réactif utilisé pour interpréter de manière appropriée les éléments d'image concernés. Il est aussi prévu que le support portant les réactifs soit un consommable à usage unique protégé de tout contact avec l'environnement extérieur par des moyens enlevés par l'utilisateur avant utilisation du dispositif. Selon les variantes de mise en oeuvre, le support portant les réactifs est monté de manière amovible et remplaçable sur lesdits moyens pour rendre l'image nette ou fait partie intégrante desdits moyens. Dans une autre variante de mise en oeuvre pour mesurer la souplesse de la peau qui est une caractéristique d'intérêt dans le domaine de la cosmétique, il est prévu que l'expression de cette caractéristique physique soit transformée en une modification de pixels dans l'image apte à en permettre l'appréciation par traitement d'image. Il est prévu par exemple d'utiliser des picots solidaires de la partie des moyens pour rendre l'image nette qui sont au contact de la peau et qui provoquent localement une déformation des lignes observables caractérisant le relief de la peau lorsqu'une force est appliquée verticalement ou en rotation. Des algorithmes de traitement d'image appropriés sont prévus pour transformer les déformations observées en une appréciation de l'élasticité de la peau. Il est aussi prévu par exemple de faire vibrer les picots de sorte à induire localement un flou dans l'image du relief de la peau dont l'étendue observable est fonction de l'élasticité de la peau. Il est à noter que dans le cas où l'appareil électronique est un Smartphone, il est avantageux de mettre en oeuvre une application capable de piloter le vibreur du téléphone qui est mécaniquement solidaire des moyens pour rendre l'image nette et ainsi pouvoir apprécier l'élasticité de la peau sans ajout de moyens matériels spécifiques.

Le système selon l'invention prévoit que les moyens pour produire un grossissement optique de l'image comprennent en outre des moyens pour permettre à l'utilisateur d'entrer au moins un élément d'information dans l'image. Il est prévu dans l'invention des moyens tels que par exemple un support sur lequel l'utilisateur peut entrer des éléments d'information dans l'image de la peau acquise par l'appareil électronique de sorte à rendre lesdits éléments d'information reconnaissables par des traitement d'image appropriés. On entend par entrer des éléments d'information par exemple noircir, cocher ou écrire dans des zones délimitées par des marquages sur ledit support. La signification zones étant identifiée par des marquages appropriés utilisant des caractères alphanumériques directement interprétables par l'utilisateur. Il est prévu que quand la place est insuffisante sur le support pour y inscrire directement la signification des zones à proximité de celles-ci, l'utilisateur se reporte à un plan imprimé sur une notice ou sur un écran pour identifier lesdites zones telles qu'elle apparaissent à la vue du support grâce à une légende qui en indique la signification. On ne sort cependant pas du cadre de l'invention dans le cas de marquages libres n'utilisant pas de zones dédiées à une signification particulière. La finalité de l'affectation de zones dédiées à la signification des marquages n'ayant pour but que la simplification des traitements d'image pour l'extraction des informations. S'agissant d'analyse de la peau, cette analyse peut être améliorée par la connaissance d'informations complémentaires telles que le sexe de la personne, son groupe ethnique, son âge etc. Certaines des informations complémentaires peuvent être considérées comme des informations sensibles, en particulier si elles peuvent être associées à des éléments d'identification de la personne tels qu'un nom un numéro de téléphone etc. Ces informations sensibles peuvent avoir un statut particulier dans le cadre de la loi de certains territoires, il est prévu dans l'invention la mise en oeuvre de moyens tels que la cryptographie et/ou l'utilisation de tiers de confiance pour échanger et/ou pour stocker l'information sensible de manière sécurisée et accessibles seulement aux entités habilitées.

Le système selon l'invention prévoit que les moyens pour produire un grossissement optique de l'image comprennent en outre des moyens pour étalonner au moins une caractéristique de l'image. Il est prévu des moyens pour inclure des éléments d'information aux caractéristiques connues dans le même plan de mise au point que la surface de peau à analyser. Ainsi la même image comprend la surface de peau à analyser et des informations connue en rapport avec l'intégrité de l'image en général et/ou en rapport avec des caractéristiques de l'image qui sont en rapport avec les caractéristiques de la peau qu'il s'agit d'analyser. Il est ainsi prévu de corriger un certain nombre d'aberrations optiques telles que les aberrations géométriques et chromatiques, le vignettage, les distorsions. Ceci permet d'utiliser dans lesdits moyens pour rendre l'image nette, des lentilles à faible coût réalisées par moulage ou encore des lentilles dites de Fresnel ayant des propriétés optiques relativement grossières. Il est aussi prévu dans l'invention d'étalonner les traitements d'images utilisés pour l'analyse de la peau. Par exemple il est prévu des témoins de couleur pour étalonner la reconnaissance de l'état des réactifs vus précédemment et/ou pour étalonner les traitements visant à déterminer la couleur de la peau c'est-à-dire la résultante colorimétrique due aux dosages de mélanine, de carotène et d'hémoglobine propres à l'échantillon de peau à analyser. L'inclusion d'éléments d'information pour étalonner la couleur est particulièrement pertinente dans les variantes de mise en oeuvre de l'invention où les moyens pour rendre l'image nette ne possèdent pas leur propre source de lumière mais utilisent une source de lumière externe dont les caractéristiques chromatiques sont inconnues. Il est aussi prévu d'analyser l'éclat de la peau c'est-à-dire sa brillance après étalonnage des traitements d'image par rapport à une pluralité de témoins de brillance connue dont l'image est acquise strictement dans les mêmes conditions que celle de l'image de la surface de peau à analyser. Concernant l'étalonnage des caractéristiques géométriques de la acquisition d'image pour les corriger par traitement d'image le cas échéant, l'invention prévoit le marquage de graduations équidistantes ayant un pas connu en X et en Y dans un demi-plan par rapport à un axe de symétrie passant par l'axe optique de l'ensemble du dispositif appareil électronique et desdits moyens pour rendre l'image nette, ledit demi-plan étant avantageusement dédié au support des éléments d'information de référence qui sont ajoutés à l'image de la surface de peau lors de l'acquisition. Par l'effet de la symétrie, la correction de toute distorsion constatée dans l'image du demi-plan comprenant les informations de référence qui sont connues, est applicable aux endroits correspondants dans le demi-plan de l'image de la peau. Ceci vaut principalement pour les distorsions géométriques, pour les altérations de la couleur, il est prévu que les corrections soient applicables en tous points de l'image. Pour corriger les altérations de la distribution de la lumière détectées en quelques points du demi-plan des informations de référence, il est prévu d'utiliser des lois de l'optique pour corriger l'image dans on ensemble. Pour mesurer le relief cutané par traitement d'image, l'invention prévoit par exemple d'exploiter la mesure de l'ombre portée des crêtes sous un éclairage incliné. Compte tenu de l'influence considérable de l'angle d'éclairage lors de l'acquisition d'image et de l'accumulation des imprécisions de tous types, seul un étalonnage image par image peut donner des résultats en Z exploitables. A cette fin, il est prévu la gravure de reliefs aux caractéristiques connues dans la zone de l'image qui est dédiée aux éléments d'informations d'étalonnage. Quelque soient les moyens pour ajouter des éléments d'information dans l'image et quelle que soit la combinaison desdits éléments d'information et leur finalité, il est prévu d'en coder la référence au moyen d'un marquage compact tel qu'un code à barres 1D ou 2D dont le décodage est réalisé par traitement d'image. L'interrogation d'une base de données à partir du code reconnu retourne la connaissance complète des caractéristiques desdits éléments d'information qui sont présents dans l'image de sorte à réaliser les traitements de correction et/ou d'étalonnage appropriés.

Le système selon l'invention prévoit que les moyens pour produire un grossissement optique de l'image comprennent en outre des moyens pour éclairer la zone de prise de vue. Il est prévu des moyens pour éclairer la surface de peau et le cas échéant des éléments d'information ajoutés dans le champ de la prise de vue. Dans les variantes les plus économiques, il s'agit de moyens d'éclairage passifs tels qu'une surface réfléchissante qui renvoie les rayons lumineux venant d'une source externe artificielle ou naturelle vers la surface dont on souhaite acquérir l'image. Il est aussi prévu dans des variantes desdits moyens pour rendre l'image nette qui sont conçues pour un modèle d'appareil électronique équipé d'un flash, un trou à l'endroit approprié pour que la lumière du flash de l'appareil puisse éclairer la surface de peau ainsi que les éléments d'information additionnels le cas échéant. Dans des variantes plus sophistiquées de mise en oeuvre de l'invention il est prévu que lesdits moyens pour rendre l'image nette comprennent une source de lumière. Il est prévu que cette source de lumière émette de la lumière blanche par le bais d'une ampoule à incandescence ou avantageusement d'une ou plusieurs diodes électroluminescentes. Il est également prévu de pouvoir éclairer la zone d'acquisition d'image en lumière monochromatique pour révéler des aspects particuliers de la peau. Parmi les différentes longueurs d'onde d'intérêt, la lumière ultraviolette ayant une longueur d'onde d'environ 365 nanomètres est particulièrement intéressante en dermatologie pour révéler des lésions et des infections cutanées. Dans le domaine de la cosmétique, une source de lumière ultraviolette permet de révéler par fluorescence des porphyrines, les pores obstrués et d'autres détails d'intérêt qui n'apparaissent pas en lumière blanche. Il est aussi prévu d'utiliser une ou plusieurs diodes lasers émettant à une longueur d'onde visible et une solution de balayage électromécanique, par exemple à micro miroir MEMS pour décrire la totalité de l'image à acquérir à partir du faisceau laser à projection ponctuelle. L'avantage d'une telle solution plus coûteuse à mettre en oeuvre étant le contraste obtenu et la possibilité d'une mesure plus précise du relief cutané. Il est également prévu des variantes de l'invention mettant en oeuvre de filtres polarisants pour améliorer le contraste et diminuer l'influence des réflexions parasites ce qui permet une capture d'image de meilleure qualité qui est propice à une meilleure exécution des traitements d'image selon l'invention.

Le système selon l'invention prévoit que les moyens pour produire un grossissement optique de l'image comprennent en outre des moyens pour déterminer la distance de mise au point par contact entre un élément matériel et une partie du corps de la personne dont on souhaite analyser au moins une caractéristique de la peau. Le système selon l'invention est prévu pour être utilisé directement par le grand public. Pour rendre l'utilisation la plus simple possible, il a été prévu de supprimer tout réglage de mise au point. Les différentes variantes de moyens pour produire un grossissement optique de l'image sont avantageusement agencées pour fixer toutes les distances ayant une influence sur la mise au point par contact entre les éléments concernés. Dans ladite première variante, deux distances ont une influence sur la mise au point, la distance entre la peau et les moyens optiques et la distance entre les moyens optiques et l'objectif de l'appareil électronique. Ces deux distances sont fixées par construction comme il apparaitra clairement dans les exemples détaillés de mise en oeuvre.

Dans ladite seconde variante, la seule distance ayant une influence sur la mise au point est la distance entre la peau et les moyens optiques.

Il est prévu dans certaines variantes, qu'une béquille ou qu'une pige solidaire des moyens pour produire un grossissement optique de l'image soit amenée au contact de la peau pour déterminer la distance d'acquisition d'image optimale. L'extrémité des éléments prévus pour entrer en contact avec la peau sont avantageusement agencés pour éviter de blesser les personnes notamment lors d'acquisition d'images dans des endroits tels que le contour de l'oeil, les paupières, la commissure des lèvres, le front, le cou.

Le système selon l'invention prévoit que les moyens pour produire un grossissement optique de l'image comprennent en outre des moyens pour apporter au moins une information à l'utilisateur. Il est prévu que l'au moins une information concerne les instructions de montage et/ou d'utilisation desdits moyens. De manière particulièrement avantageuse, il est prévu que l'au moins une information soit en rapport avec la destination des données produites par l'appareil électronique grand public. Il s'agit par exemple du numéro de téléphone d'un serveur apte à recevoir des MMS, une adresse de courrier électronique apte à recevoir des fichiers d'image en pièce jointe, une adresse de serveur ftp ou de site web apte à permettre le chargement d'images sur un serveur, une adresse de courrier physique permettant l'envoi par voie postale d'au moins une impression d'images ou de support matériel contenant des données codant au moins une image.

Le système selon l'invention prévoit que les moyens pour produire un grossissement optique de l'image sont agencés pour être livrés aux utilisateurs sous une forme de faible épaisseur utilisant des matériaux flexibles de sorte à pouvoir par exemple être encartés dans un magazine ou envoyés sous enveloppe. Dans le cadre d'une utilisation grand public de l'invention dans le domaine de la cosmétique, il est particulièrement préféré d'agencer lesdits moyens pour rendre l'image nette de sorte que leur coût de réalisation et de distribution soit le plus faible possible sachant qu'une fois l'au moins une caractéristiques de la peau d'une personne donnée analysée, lesdits moyens pour rendre l'image nette n'ont plus d'utilité. Le fait que ces moyens selon l'invention, dans ce contexte d'utilisation, sont à usage unique permet de mettre en oeuvre des techniques de réalisation peu couteuses. Ces techniques de réalisation peu couteuses qui sont en outre aptes à une distribution de masse à faible coût, sont particulièrement compatibles avec l'utilisation de moyens pour transformer l'expression d'au moins une caractéristique chimique de la peau de sorte à permettre une appréciation de l'au moins une caractéristique chimique de la peau par traitement d'image tels que décrits précédemment. Ces moyens utilisant la chimie étant généralement disposés sur un support plan protégé, de l'air et de l'humidité dans un emballage de protection adéquat, l'ensemble formant une solution de faible épaisseur et de surcroit relativement flexible.

Dans des variantes encore plus préférées, lesdits moyens pour rendre l'image nette sont réalisés par prédécoupe à l'emporte pièce dans une feuille d'un matériau flexible tel qu'un carton ou une matière plastique avantageusement biodégradable ou recyclable. Les moyens optiques nécessaires peuvent être réalisés dans une matière rigide compte tenu de leur petite surface et de leur épaisseur qui peut rester compatibles avec des contraintes d'encartage et qui n'affecte pas le caractère globalement flexible de la solution proposée. Il est également prévu d'utiliser des moyens optiques utilisant des lentilles dites de Fresnel pour réaliser des lentilles flexibles de très faible épaisseur eu égard à leurs propriétés optiques. Les variantes de mise en oeuvre de l'invention visant une distribution à plat sont prévues pour un assemblage final réalisé par l'utilisateur avant de procéder à l'analyse. Ainsi les moyens pour rendre l'image nette seront avantageusement agencés pour en simplifier le montage final. Les instructions de montage utilisant autant que possible des dessins seront avantageusement imprimées sur la feuille qui est à la base de la réalisation desdits moyens pour rendre l'image nette. Des solutions fondées sur le pliage sont prévues pour simplifier le montage. Ce dernier comprenant le détachement de la forme livrée à plat de son du support le cas échéant, son dépliage pour reconstituer les moyens pour rendre l'image nette dans leur volume apte à leur utilisation. Une étape complémentaire de verrouillage en position déployée desdits moyens peut être rendue nécessaire selon les variantes. Il peut s'agir par exemple de l'insertion d'au moins une languette dans une fente prévue à cet effet ou de la mise en oeuvre d'un adhésif. L'invention prévoit aussi un procédé pour analyser au moins une caractéristique de la peau par des moyens relevant de l'optique et du traitement d'image. Le procédé selon l'invention comprend des étapes au cours desquelles :
- On acquière au moins une image numérique comprenant une surface de peau au moyen d'un appareil électronique grand public;
- On récupère des données en relation avec l'au moins une image acquise dans un format apte aux traitements d'image;
- On traite les données récupérées qui sont en relation avec l'image acquise de sorte à produire au moins une information en rapport avec au moins une caractéristique de la peau;

La première étape concerne l'acquisition d'image proprement dite. Il est prévu que le contenu de cette étape varie selon les options de mise en oeuvre de l'invention et les directives associées à l'application qui sont indiquées à l'utilisateur. Il est plus particulièrement prévu que l'étape d'acquisition d'image comprenne plusieurs sous étapes de prises de vues de zones de peau spécifiques. Cette étape est exécutée en utilisant un appareil équipé d'un capteur d'image.

La seconde étape vise la récupération des données en relation avec l'au moins une image acquise, dans un format standard supporté par l'appareil électronique qui est apte au traitement d'image. Cette étape est le plus souvent exécutée dans l'appareil ayant été utilisé pour l'étape d'acquisition de l'image mais il est prévu qu'elle puisse être exécutée dans un ou plusieurs autres appareils tels qu'un ordinateur ou une tablette numérique.

La troisième étape est celle du traitement des données récupérées qui sont en relation avec l'image acquise de sorte à produire au moins une information d'intérêt en rapport avec l'au moins une caractéristique de la peau analysée. Selon les variantes de mise en oeuvre, cette étape est exécutée en totalité dans au moins un serveur distant ou elle est au moins en partie exécutée dans l'appareil électronique avant transmission des données à l'au moins un serveur distant pour finaliser les traitements, le cas échéant pour stocker les données en relation avec l'au moins une image et/ou pour accéder à au moins une base de données et/ou pour retourner des informations à l'utilisateur.

Le procédé selon l'invention prévoit en outre que l'au moins une image numérique comprenant une surface de peau acquise au moyen d'un appareil électronique grand public contient la tête de la personne dont on souhaite analyser au moins une caractéristique de la peau. L'image de la tête humaine est particulièrement riche en informations. Les informations que l'invention prévoit d'extraire d'images de la tête des personnes sont par exemple le type ethnique, le sexe, le type de cheveux, l'appartenance à une tranche d'âge etc.

Il est prévu dans l'invention des traitements d'images pour extraire automatiquement tout ou partie d' informations d'intérêt aux fins de renseigner une base de données et/ou pour affiner ou compléter l'analyse d'au moins une caractéristique de la peau par recours à des informations stockées dans au moins une base de données. Ceci à partir de la connaissance d'au moins une information extraite de l'image de la tête de la personne.

Le procédé selon l'invention comprend en outre une étape au cours de laquelle :
- On place des moyens pour produire un grossissement optique de l'image préalablement à son acquisition entre l'objectif dudit appareil électronique grand public et la peau dont on souhaite analyser au moins une caractéristique.

L'acquisition de l'image d'une partie du corps représentant une surface relativement importante de peau en vue d'un premier niveau d'analyse et possible directement avec des appareils électroniques grand public. En dehors de l'acquisition déjà évoquée de l'image de la tête de la personne qui présente un grand intérêt et qui est possible avec des appareils électroniques standards sans accessoire, l'analyse directe d'au moins une caractéristique de la peau requière des caractéristiques d'acquisition d'image au moins de type macrophotographie.

Les appareils électroniques grand public courants équipés d'une fonction caméra ou appareil photo n'offrent pas toujours les caractéristiques requises c'est pourquoi l'invention prévoit l'utilisation de moyens pour rendre l'image nette lors d'une acquisition d'image à une distance plus courte que la plus courte distance possible pour laquelle ledit appareil électronique grand public a été conçu. Une étape utilisant de tels moyens selon l'invention est prévue aux fins d'en analyser au moins une caractéristique et/ou pour examiner un grain de beauté ou une lésion en rapport avec le cancer de la peau.

Le procédé selon l'invention comprend en outre une étape au cours de laquelle :
- On entre au moins une information dans l'au moins une image de la peau telle qu'elle sera acquise au moyen d'un appareil électronique grand public.

Cette étape vise à permettre à l'utilisateur d'entrer des informations dans des champs prévus à cet effet compris dans l'image à acquérir au moyen d'un appareil électronique. Il est prévu d'entrer des informations explicites telles que l'âge ou la tranche d'âge, le sexe, l'appartenance à un groupe ou à un sous-groupe ethnique renvoyant à des caractéristiques de la peau statistiquement connues comme pas exemple pour le sous-groupe à « peau de roux » du groupe ethnique caucasien. L'entrée d'informations est prévue sous la forme d'un marquage à au moins un endroit correspondant à au moins un choix dans une proposition identifiée et/ou sous la forme d'inscription directe d'informations utilisant des caractères alphanumériques, par exemple pour entrer des informations d'identification et/ou d'adressage de la personne dont la peau est analysée ou à qui les retours doivent être transmis s'il ne s'agit pas de la même personne. L'intérêt principal de cette étape est d'inclure des informations d'intérêt dans chaque image de manière indissociable de l'image d'une surface de peau proprement dite. Des traitements d'images appropriés sont utilisés pour extraire les informations ainsi introduites dans l'image de sorte à pouvoir les utiliser dans le système de traitement de l'information associé à la mise en oeuvre de l'invention. Il est prévu dans certaines variantes de mise en oeuvre, de ne stocker les images qu'après extraction des informations incluses par l'utilisateur et après une étape complémentaire d'effacement d'au moins les pixels correspondant aux informations incluses par l'utilisateur. Certaines variantes de l'invention prévoient en outre de continuer de lier les informations ajoutées par l'utilisateur à une image, non plus sous forme brute de pixels dans cette image mais sous une forme décodée et structurée en métadonnées associées à l'image, ces informations étant comprises dans le fichier associé à l'image numérique.

Le procédé selon l'invention comprend en outre une étape au cours de laquelle :
- On corrige, l'au moins une image de la peau telle qu'acquise au moyen d'un appareil électronique grand public, et/ou les données issues du traitement de l'au moins une image, en fonction d'informations d'étalonnage qui sont introduites dans l'image par les moyens pour produire un grossissement optique de l'image.

Cette étape vise le cas échéant à corriger des défauts de l'image qui sont par exemple dus à l'utilisation de moyens optiques et ou mécaniques de faible qualité pour réaliser lesdits moyens pour rendre l'image nette à moindre coût et/ou sous une forme livrable à plat. Cette étape permet en outre de calibrer tout ou partie des caractéristiques des données en relation avec l'image acquise de sorte à fortement réduire, voire à éliminer l'influence de l'hétérogénéité des solutions d'acquisition d'images numériques mises en oeuvre dans les appareils électronique grand public, de leurs caractéristiques et de leurs performances. Il est prévu dans certaine variantes de mise en oeuvre de l'invention que cette étape comprenne des traitements d'image visant à normaliser les images avant les traitements pour l'analyse proprement dite. On entend par normaliser par exemple, recadrer, remettre à l'échelle, effectuer au moins une rotation, corriger les couleurs etc. de sorte à permettre la comparaison de plusieurs acquisitions d'images d'une même surface de peau d'intérêt faites à des instants différents et possiblement avec des appareils différents. Les traitements de normalisation sont dans certains cas de mise en oeuvre, une étape de préparation des images avant l'exécution des traitements en vue de l'analyse d'au moins une caractéristique de la peau, la préparation des images améliorant l'efficacité des traitements d'analyse.

Le procédé selon l'invention comprend en outre une étape au cours de laquelle :
- On rapproche les informations de localisation d'une réaction cutanée visible dans une image, la connaissance de l'endroit correspondant sur un support d'application de la substance ayant provoqué la réaction cutanée et la connaissance de la nature de la substance qui était déposée à l'endroit correspondant sur le support d'application de sorte à produire au moins une information en rapport avec la nature de la substance ayant provoqué la réaction cutanée.

On entend par réaction cutanée dans le contexte de l'invention, toute manifestation visible dans une image qui est circonscrite à une localisation précise et qui apparait contrastée par rapport à la surface de peau qui constitue le fond de l'image. Une réaction cutanée est visible à l'oeil nu et dans l'image acquise par un appareil électronique par un changement de couleur et/ou par un changement de relief.

Le procédé selon l'invention comprend en outre une étape au cours de laquelle :
- On reçoit d'un serveur une liste d'au moins un produit à éviter en ce qu'il contient une substance identifiée à l'étape précédente et/ou une liste de produits recommandés parce qu'ils ne contiennent pas une substance identifiée à l'étape précédente, lesdits produits étant identifiés par leur marques commerciales en usage sur un territoire donné de sorte à guider l'utilisateur dans ses achats.

Avantageusement les références desdits produits à éviter ou recommandés seront accessibles par thèmes en rapport avec la vie quotidienne et/ou regroupés en rapport avec la manière dont on peut se les procurer, par exemple en regroupant les produits susceptibles de se trouver dans un même rayon ou dans un même point de vente spécialisé. Il est aussi prévu de modifier l'ordre d'apparition des produits dans les listes en fonction de notations ou de classements issus de retour des utilisateurs ou encore en fonction d'achat de position dans les liste par des marques ou des marchands.

Le procédé selon l'invention comprend en outre une étape au cours de laquelle :
- Une entité marchande propose à la vente des produits appartenant à la catégorie des produits recommandés à l'étape précédente.

Le procédé selon l'invention comprend en outre une étape au cours de laquelle :
- On sélectionne au moins un produit parmi les produits proposés à la vente à l'étape précédente.

Le procédé selon l'invention comprend en outre une étape au cours de laquelle :
- On mémorise les préférences de l'utilisateur à travers les choix qu'il fait dans les propositions qui lui sont faites. Les dites préférences associées à l'utilisateur dans une base de données étant possiblement utilisées à des fins promotionnelles et/ou pour pousser de l'information d'intérêt et/ou pour envoyer des échantillons de produits susceptibles de plaire après essai et/ou pour proposer d'autres produits susceptibles de plaire d'après l'au moins un produit sélectionné à l'étape précédente.

Le procédé selon l'invention comprend en outre une étape au cours de laquelle :
- On valide une transaction relative au transfert de propriété de l'au moins un produit finalement sélectionné.

Le procédé selon l'invention comprend en outre une étape au cours de laquelle :
- On pose au moins une question à l'utilisateur.

Cette étape vise à renseigner des informations nécessaires au système le cas échéant, par exemple lorsqu'il n'est pas possible ou pas souhaitable d'obtenir cette information automatiquement par traitement d'image. L'invention prévoit selon les variantes de mise en oeuvre que tous les moyens connus pour interagir avec l'utilisateur puissent être utilisés à cette fin, par exemple par échange de SMS, de courriels, de questions et de réponses sur un site web, par l'intermédiaire d'une application dédiée exécutée sur un Smartphone, sur une tablette numérique, sur un téléviseur connecté à Internet, dans une Set Top Box (boitier adaptateur) raccordé à un téléviseur etc.

Le procédé selon l'invention comprend en outre une étape au cours de laquelle :
- On transmet au moins une information à un serveur distant à partir d'un appareil grand public connecté à un réseau.

Cette étape permet de transférer le fichier correspondant à l'au moins une image brute issu de l'appareil électronique grand public à au moins un serveur distant dans lequel des traitements de l'image sont exécutés pour analyser au moins une caractéristique de la peau. Une architecture de système de type « cloud » est particulièrement appropriée pour pouvoir adapter automatiquement les moyens de traitements distants à la demande ou pour dé-ballaster un excédent de charge de traitement dans une architecture client serveur classique. Selon les variantes de mise en oeuvre, il est prévu que tout ou partie des traitements selon l'invention soient exécutés sur l'au moins un serveur selon que les appareils grand public utilisés sont respectivement inaptes ou en capacité d'exécuter localement certains des traitements selon l'invention.

Le procédé selon l'invention comprend en outre une étape au cours de laquelle :
- On reçoit au moins une information venant d'au moins un serveur distant.

Cette étape vise à remettre à l'utilisateur au moins une partie des résultats attendus après mise en oeuvre du procédé selon l'invention. La nature et le contenu de l'au moins une information reçue dépend de l'utilisation du procédé selon l'invention et des moyens utilisés pour la recevoir. Dans le cas d'une utilisation dans le domaine de la cosmétique, l'utilisateur reçoit par exemple une proposition de produits adaptés aux caractéristiques de sa peau et/ou aux préférences qu'il a pu communiquer au système d'information. Généralement l'étape de réception d'au moins une information dans le domaine de la cosmétique est suivie d'étapes de sélection de produits, de prise de commande et de paiement.

Dans le cas d'une utilisation dans le domaine de la dermatologie et plus particulièrement pour la détection précoce du cancer de la peau, l'utilisateur reçoit par exemple une information indiquant qu'aucun problème n'a été détecté ou une invitation à consulter rapidement un dermatologue en cas de détection d'une anomalie ou en cas de doute. Généralement l'étape de réception d'au moins une information dans le domaine de la dermatologie est suivie d'étapes de fourniture d'une listes de dermatologues ou d'hôpitaux comprenant un service de dermatologie à proximité de l'utilisateur préalablement localisé, et le cas échéant, d'une étape de prise de rendez-vous automatisée avec avantageusement une gestion de priorité dépendant de l'urgence du cas.

Il est prévu d'utiliser le procédé selon l'invention pour proposer au moins un produit adapté à la peau et/ou par extension aux cheveux de la personne dont on a analysé au moins une caractéristique de la peau. En effet, la variété en augmentation constante et la segmentation de plus en plus fine de l'offre de produits cosmétique en fonction des caractéristiques de la peau, et par extension des cheveux, rend de plus en plus difficile le choix pour l'utilisateur des produits qui lui conviennent. En outre, pour les professionnels de la cosmétique, de la production aux points de vente, la multiplication des références augmente la complexité et les coûts associés à la logistique et à la commercialisation. L'invention vise non seulement à simplifier la sélection de produits cosmétiques à l'intérieur des gammes traditionnelles mais il est prévu qu'elle permette de multiplier sans limite le nombre de combinaisons entre substances actives et bases cosmétiques. Il est également prévu que le couplage de l'invention avec un système de prise de commande et de vente à distance ainsi qu'avec un système de gestion de production approprié permettent la fabrication de produits cosmétiques, quasiment sur mesure, et possiblement après leur prise de commande.

Le procédé selon l'invention comprend en outre une étape au cours de laquelle :
- On sélectionne au moins un produit dans un ensemble d'au moins un produit cosmétique proposé comme étant adapté à l'au moins une caractéristique de la peau qui a été analysée ou en rapport avec celle-ci.

On entend par proposition en rapport avec l'au moins une caractéristique de la peau analysée, l'existence d'au moins une relation indirecte comme par exemple la proposition de produits de traitement des cheveux crépus après avoir détecté une peau de type africain par la mise en oeuvre de l'invention. Le procédé selon l'invention comprend en outre une étape au cours de laquelle :
- On mémorise les préférences de l'utilisateur à travers les choix qu'il fait dans les propositions qui lui sont faites. Les dites préférences associées à l'utilisateur dans une base de données étant possiblement utilisées à des fins promotionnelles et/ou pour pousser de l'information d'intérêt et/ou pour envoyer des échantillons de produits susceptibles de plaire après essai et/ou pour proposer d'autres produits susceptibles de plaire d'après l'au moins un produit sélectionné à l'étape précédente.

Le procédé selon l'invention comprend en outre une étape au cours de laquelle :
- On valide une transaction relative au transfert de propriété de l'au moins un produit finalement sélectionné.

Il est prévu de manière avantageuse que l'étape de sélection et l'étape de la transaction proprement dite et/ou de sa validation soit proposée à l'utilisateur par le biais de l'appareil électronique grand public utilisé pour analyser la peau. Par exemple cette étape peut être mise en oeuvre en utilisant un téléphone mobile simple qui se connecte à un serveur vocal approprié ou à une personne. Cette étape peut aussi être mise en oeuvre en utilisant un téléphone simple à partir duquel on échange des SMS ou des MMS. Un Smartphone ou une tablette communicante munie d'une camera offre en outre la possibilité d'une mise en oeuvre de cette étape par le biais d'une application dédiée téléchargeable. Cela étant on ne sort pas du cadre de l'invention si un autre moyen est utilisé pour ces étapes, par exemple l'accès à un site marchand au moyen d'un navigateur web générique ou par une application spécifique exécuté sur un microordinateur, sur une tablette ou sur un téléviseur connecté à Internet.

Le procédé selon l'invention comprend en outre une étape au cours de laquelle :
- On crédite un compte de fidélité selon des modalités et des bénéfices associés décrits dans un ou plusieurs supports d'information publics.

Le procédé selon l'invention comprend en outre une étape au cours de laquelle :
- On lance la fabrication de l'au moins un produit sélectionné.

Les avantages de l'inventions dans ce contextes sont multiples, outre la proposition de produits en parfaite adéquation avec les caractéristiques de la peau et ceci sans peine pour l'utilisateur, puis la sélection et l'acquisition d'au moins un produit parmi ceux proposés, le lancement de la production après ces étapes permet en outre de réduire ou de supprimer les conservateurs dans la composition des produits. La suppression des temps de stockage longs dans les circuits de production et de distribution rendant les conservateurs moins nécessaires ou inutiles. Par ailleurs les gains économiques qui résultent de la suppression des temps de stockage longs que permet l'invention sont significatifs.

Il est prévu d'utiliser le procédé selon l'invention pour détecter un cancer de la peau chez la personne dont on a analysé au moins une caractéristique de la peau. La détection précoce du cancer de la peau augmente les chances de guérison. En effet, il est connu que le cancer de la peau peut avoir un bon pronostic de guérison s'il est traité à temps mais qu'il s'agit d'un type cancer dangereux en ce qu'il susceptible d'évoluer rapidement et peut dans certains cas passer au stade métastatique en quelques mois. L'invention est particulièrement pertinente pour détecter précocement le cancer de la peau en ce qu'elle permet notamment aux personnes qui se savent à risque comme les personnes à peau de roux ou celles ayant eu de forts coups de soleil dans leur enfance, de se surveiller aussi souvent qu'elles le souhaitent, de manière libre, économique et efficace. En outre l'invention permet de faire face au double problème de la progression constante du nombre de mélanomes qui apparaissent dans les populations, avec par exemple une progression de 5 à 7% du nombre de mélanomes diagnostiquées par an en Europe, et la relative pénurie de dermatologues. L'invention permet d'améliorer la fréquence et donc la précocité de la détection du mélanome sans saturer les capacités de consultation mais au contraire en orientant les cas douteux et les détections avérés vers les spécialistes pour une prise en charge rapide et efficace tant pour le patient que pour le médecin.

Il est prévu que les traitements informatiques selon l'invention, appliqués à l'image d'un grain de beauté ou d'une lésion cutanée suspecte, soient optimisés pour détecter des lésions :
De couleur noire,
- Asymétrique,
- A bords irréguliers,
- A coloration non homogène,
- Ayant un diamètre important (> 6 mm),
- Évolutives.

Pour cette dernière catégorie, il est prévu que le système selon l'invention intègre des moyens pour mémoriser une pluralité d'images horodatées de la lésion qui est sous surveillance. Les images étant acquises à des intervalles réguliers dans le cadre d'une bonne prévention. Il est prévu que le système selon l'invention sollicite automatiquement tout utilisateur déjà enregistré, par l'envoi automatique d'un message électronique, avec des rappels le cas échéant, lorsqu'il serait souhaitable de faire une nouvelle acquisition parce qu'un temps trop long s'est écoulé depuis la dernière acquisition. Des traitements d'images sont en outre prévus pour recaler les images unitaires, par exemple en rotation, en translation dans le plan de l'image, sur le plan de l'échelle, en vue d'en permettre la comparaison automatique.

Il est aussi prévu d'utiliser le procédé selon l'invention pour détecter la sensibilité d'une personne à une ou plusieurs substances allergisantes. Il est communément constaté que le nombre de substances allergisantes ne cesse d'augmenter ainsi que la sensibilité des personnes. Outre la gêne pour les personnes concernées qu'il convient de réduire au moins en leur permettant de connaitre les substances auxquelles elles doivent éviter de s'exposer, les allergies deviennent un enjeu de santé publique compte tenu de l'importance du phénomène. Rechercher la cause d'une allergie avec les moyens à l'état de l'art peut être longue et fastidieuse. L'invention permet de faciliter et d'accélérer l'obtention des résultats lors de la recherche de la cause d'une allergie. Les solutions de l'invention permettent en outre au grand public de procéder lui-même à cette recherche ou au moins à une recherche préliminaire visant à resserrer le champ de la recherche que finalisera un médecin allergologue. L'information en rapport avec au moins une caractéristique de la peau est binaire dans cette application. Elle repose sur la détection de la présence ou de l'absence d'une réaction cutanée visible par un changement de couleur et/ou par un changement de relief cutané localisé à l'endroit de la mise en contact avec la substance allergisante. Cette information est exploitée dans une étape complémentaire de rapprochement entre la localisation de la réaction cutanée dans l'image, la connaissance de l'endroit correspondant sur le support d'application de la substance allergisante et de la connaissance de la nature de la substance qui était déposée à cet endroit sur le support.

L'invention utilisée pour la recherche de substances allergisantes repose sur l'utilisation de consommables spécifiques qui sont différents de ceux décrits précédemment dans le cadre des applications à la cosmétique et à la détection du cancer de la peau. Le consommable selon l'invention pour la recherche de substances allergisantes comprend un support sur la surface duquel est déposé une pluralité de substances possiblement allergisantes. Les dépôts de substances étant avantageusement organisés en matrice au sein d'une grille régulière matérialisée par des moyens visibles. Une grille apparaissant de manière très contrastée dans l'image permet de réaliser des consommable ayant une grande densité sans risque d'erreur de lecture. A cette fin, il est avantageux de matérialiser un encadrement d'un ou de plusieurs sites de dépôt de substance à la fois par un marquage de couleur foncée et un marquage de couleur claire de sorte que quelle que soit la couleur de la peau de la personne, il se trouve toujours des repères visibles dans l'image par rapport à la couleur de fond de la peau. La surface supportant la pluralité de substances allergisantes comprend avantageusement autant de moyens pour occasionner une microlésion de la peau que de substances, de sorte à mettre lesdites substances en contact avec les tissus placés sous la barrière de la peau et qui sont accessibles au système immunitaire lors de l'étape d'application du consommable selon l'invention sur une surface de peau. Les moyens pour occasionner les microlésions sont de préférence agencés pour que l'application reste indolore. Le mode de réalisation préféré est un substrat constitué d'une plaque de matière plastique flexible de faible épaisseur dont une face est structurée pour former sur chaque site prévu pour recevoir un produit possiblement allergisant, une ou plusieurs micropointes ou microlames agencées pour qu'une pression modérée exercée avec les doigts ou par l'intermédiaire d'un accessoire prévu à cet effet sur l'autre face provoque les microlésions nécessaire à la mise en contact de chaque substance dont l'effet est à tester et du système immunitaire. Il est prévu en outre que le consommable selon l'invention puisse être en partie retiré après une période suffisante d'exposition du système immunitaire aux substances à tester pour l'étape d'acquisition de l'image selon l'invention. La partie minimale du consommable à retirer pour cette étape est celle qui masque les points où le système immunitaire a été exposé aux substances. La partie maximale du consommable à retirer pour cette étape est celle qui laisse subsister de manière visible lors de l'étape d'acquisition de l'image :
- des éléments suffisants de la structure selon laquelle les points de mise en contact des substances étaient organisés pour pouvoir déterminer par traitement d'image et/ou par calcul la position de chaque point de mise en contact, et
- des moyens pour identifier le consommable de sorte à permettre aux traitements d'image de requérir dans une base de données l'ensemble de ses caractéristiques nécessaires à l'analyse.

Après l'étape d'acquisition de l'image, les éléments résiduels du consommable peuvent être retirés de la peau. L'analyse par traitement d'image selon l'invention repose sur la détection des points ayant entrainé une réaction du système immunitaire et sur l'identification d'une ou plusieurs substances ayant entrainée la réaction par leur localisation dans l'image et par la correspondance entre la localisation des points dans l'image et les substances mises en contact connaissant les caractéristiques du consommable au regard de la nature des substances qu'il comprend et de leur localisations respectives.

### Brève description des dessins

D'autres avantages et caractéristiques de l'invention apparaîtront à l'examen de la description détaillée de modes de mise en oeuvre nullement limitatifs, et des dessins annexés où,
Selon le premier aspect de l'invention :
La figure 1 illustre l'architecture globale du système selon l'invention.
La figure 2 illustre une première variante de moyens de grossissement.
La figure 3 illustre l'architecture interne des moyens de la figure 2.
La figure 4 illustre les moyens de fixation de l'exemple de la figure 2.
La figure 5 illustre une première variante livrable à plat, face externe.
La figure 6 illustre la face interne de la variante à plat de la figure 5.
La figure 7 illustre la variante à plat de la figure 5 en volume.
La figure 8 illustre une sous-variante de la variante de la figure 5.
La figure 9 illustre la variante à plat de la figure 5 en situation opérationnelle.
La figure 10 illustre une autre sous-variante de la variante de la figure 5.
La figure 11 illustre une seconde variante livrable à plat, face externe.
La figure 12 illustre la variante à plat de la figure 11 en situation.
La figure 13 illustre des moyens consommables pour la seconde variante.
La figure 14 illustre des moyens pour étalonner les mesures et évaluations.
La figure 15 illustre une autre disposition des moyens de la figure 14.
La figure 16 illustre l'utilisation de deux axes de symétrie.
La figure 17 illustre une variante de consommable libérant la zone centrale.
La figure 18 illustre des moyens pour entrer des d'informations dans l'image.
La figure 19 illustre les moyens de la figure 18 avec un consommable.
La figure 20 illustre des traitements d'image pour corriger des distorsions.
La figure 21 illustre des traitements pour étalonner, corriger et transformer
La figure 22 illustre une utilisation pour la détection du cancer de la peau.
La figure 23 illustre un raffinement de la première variante du dispositif.
La figure 24 illustre le raffinement de la figure 23 pour la seconde variante.
La figure 25 illustre la détection par traitements sur images isolées.
La figure 26 illustre la détection par traitements sur images multiples.
La figure 27 illustre un consommable pour substances allergisantes.
La figure 28 illustre la pose du consommable pour substances allergisantes.

### Description détaillée des figures et des modes de réalisation

D'autres particularités et avantages de l'invention apparaîtront encore dans la description ci-après. Aux dessins annexés donnés à titre d'exemples non limitatifs :

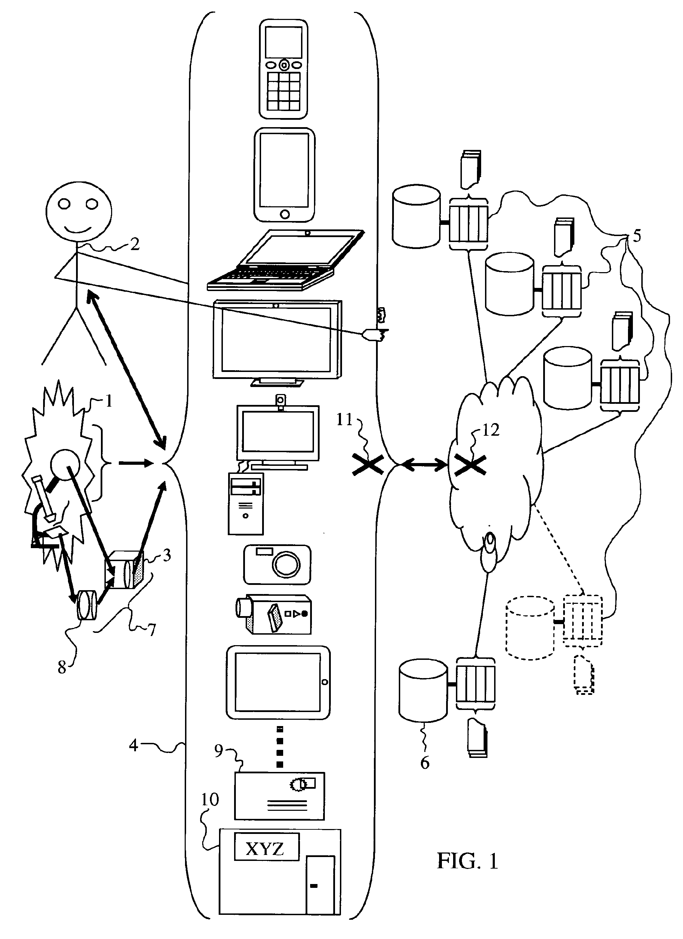

La **figure 1** illustre l'architecture globale du système selon l'invention. Au moins une caractéristique de la peau **1** d'une personne, qui peut être l'utilisateur **2** lui même ou une tierce personne, est à analyser par des moyens relevant principalement de l'optique **(3, 4)** et du traitement d'image **(4, 5).** Il est prévu que d'autres techniques complètent ces moyens pour notamment de s'accommoder d'imperfections des matériels grand public **4** utilisés et/ou de la limitation des connaissances dans le domaine de la peau de la population générale qui est principalement visée par l'invention. Les techniques complémentaires prévues sont par exemple la réponse à des questions explicites et/ou le recours à des modèles statistiques et/ou à l'interrogation de bases de données **6** en rapport avec la génétique.

Selon les caractéristiques de la peau que l'on souhaite analyser, sachant qu'a partir de quelques caractéristiques analysées, d'autres peuvent être déduites par recours aux techniques complémentaires précitées, l'invention prévoit d'utiliser un ou plusieurs appareils électroniques grand public **4** pour acquérir une ou plusieurs images et pour transmettre les données correspondantes aux moyens de traitements **(4, 5).** L'invention prévoit en outre que l'acquisition d'image d'une surface de peau soit faite directement à partir d'un appareil électronique grand public **4** et/ou en utilisant des moyens **7** pour produire un grossissement optique de l'image. Ces moyens comprenant le cas échéant plusieurs sous ensembles **(3, 8)** pour obtenir le grossissement nécessaire en particulier pour analyser les cheveux.
L'invention prévoit en outre d'étendre la notion de moyen électroniques grand public à tous moyens accessibles au grand public, par exemple des automates pour réaliser des photos d'identités. En ce qui concerne la transmission des informations produites à des moyens de traitement distants, outre l'utilisation des moyens préférés relevant des télécommunications et de la connexion d'appareils électroniques grand public à des réseaux, il est aussi prévu d'utiliser des réseaux de transports de l'information sur des supports physiques tels que le courrier **9** ou l'apport de l'information en des lieux physiques **10** déterminés en fonction du domaine d'utilisation de l'invention. De tels lieux sont par exemple des instituts de beauté dans le domaine de la cosmétique ou des cabinets médicaux dans le domaine de la dermatologie.
Il est prévu selon les variantes de mise en oeuvre de l'invention que les informations produite à transmettre à des moyens distants soient des informations brutes directement issue de l'étape d'acquisition d'image, des informations partiellement traitées ou traitées en totalité par des moyens relevant de l'électronique grand public qui sont à la disposition de l'utilisateur.
Bien entendu, les moyens utilisables pour transmettre l'information dans le sens utilisateur vers systèmes de traitement de l'information extérieurs sont aussi utilisables pour transmettre des informations et/ou des biens et/ou des services à l'utilisateur à partir d'entités distantes. Il est prévu en outre que les moyens **4** accessibles au grand public utilisés pour produire des informations soient différents de ceux utilisés pour en recevoir, c'est ce que symbolise la croix **11.** Il est aussi prévu d'utiliser une pluralité de réseaux interconnectés pour transmettre des informations entre les moyens mis en oeuvre, c'est ce que symbolise la croix **12.** Par exemple l'utilisateur **2** peut acquérir une image de la peau **1** avec son téléphone portable, envoyer le fichier correspondant par MMS ou en pièce jointe à un courriel par l'intermédiaire du réseau téléphonique cellulaire, l'information est reçue par un serveur par l'intermédiaire d'un réseau IP interconnecté avec le réseau de téléphonie cellulaire. Par ailleurs, des transmissions de données via des réseaux interconnectés sont aussi prévues du côté serveur. Il est prévu qu'une pluralité de serveurs interconnectés **5** partagent de la puissance de calcul et des moyens de stockage dans le cadre d'une architecture distribuée de type « cloud computing » (en langue anglaise). Il est aussi prévu que les serveurs **5** transmettent des requêtes à des bases de données distantes **6** également interconnectées. L'information en retour et/ou les biens et/ou les services associés à l'analyse de la peau selon l'invention sont mis à disposition de l'utilisateur par au moins un moyen accessible au grand public **4.** Ces moyens étant principalement des moyens électroniques connectés à un réseau mais aussi possiblement des moyens d'acheminement ou de mise à disposition physiques.

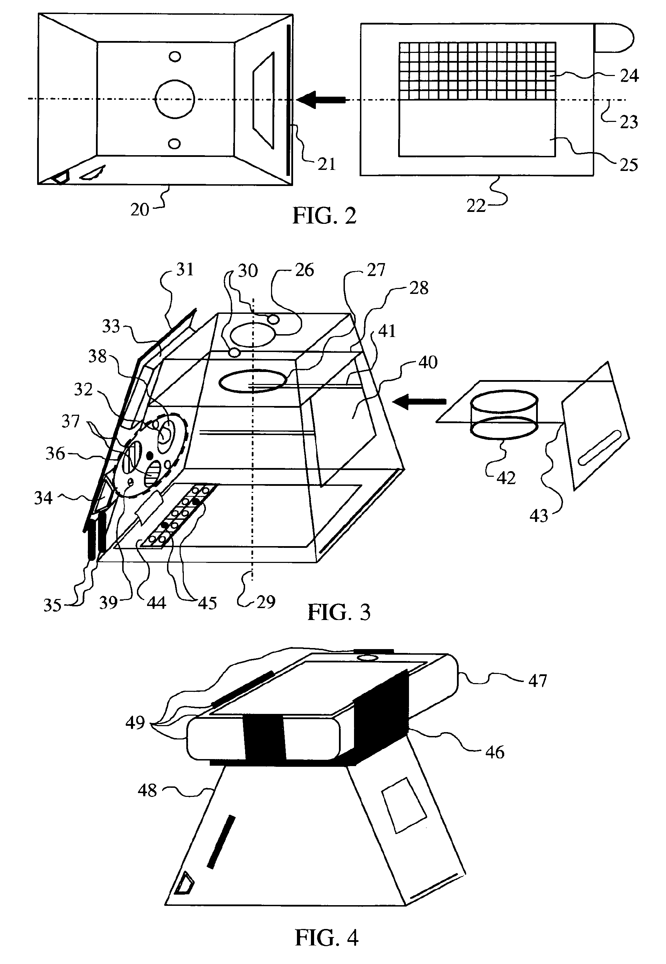

La **figure 2** illustre une première variante de moyens pour produire un grossissement optique de l'image. Cette première variante donne un exemple de mise en oeuvre desdits moyens pour rendre l'image nette selon l'invention sous la forme d'un objet qualitatif et durable apte à être utilisée par le grand public mais aussi par des professionnels. Les solutions techniques utilisée dans cet exemple non limitatif sont un boitier **20** en matière plastique injectée rigide dans lequel prennent place un sous-ensemble électronique et les moyens optiques pour rendre l'image nette à une distance plus courte que la plus courte pour laquelle l'appareil grand public utilisé pour acquérir l'image a été conçu. Une lentille convexe en verre ou en matière plastique de qualité optique est utilisée à cette fin. Un assemblage de lentilles est aussi prévu pour des variantes très qualitatives où la réduction des distorsions et des aberrations optiques à la source est recherchée pour par exemple réduire les besoins de traitements d'image et réduire les temps de réponse du système. Des moyens **21** sont prévus pour introduire et pour guider un sous-ensemble **22** placé dans le plan de l'image de la peau à acquérir. Ledit sous-ensemble est agencé pour transformer l'expression d'au moins une caractéristique physique, chimique ou biologique de la peau de sorte à permettre une appréciation de l'au moins une caractéristique physique, chimique ou biologique de la peau par traitement d'image et/ou des moyens pour permettre à l'utilisateur d'entrer au moins un élément d'information dans l'image et/ou pour étalonner au moins une caractéristique de l'image. Ledit sous-ensemble exploite la symétrie des moyens optiques mais aussi avantageusement la symétrie dans la distribution de la lumière sur la surface dont on souhaite acquérir l'image. Dans l'exemple de la **figure 2****,** une symétrie existe de part et d'autre de l'axe **23** pour les moyens optiques et pour les moyens d'éclairage. Ainsi dans l'exemple de la **figure 2****,** la réponse du système complet, lors de l'acquisition de l'image comprise dans le demi-plan **24** pour chaque point est équivalente à la réponse pour le point symétrique dans le demi-plan **25.** Les traitements de corrections et/ou d'étalonnage selon l'invention reposent sur la connaissance des caractéristiques de l'image d'un demi-plan de référence **24** puis sur la correction des caractéristiques de pixels, ou de blocs de pixels, du demi-plan de référence de l'image acquise pour obtenir le résultat préalablement connu. Ensuite les mêmes corrections sont appliquées aux pixels, ou aux blocs de pixels, symétriques du demi-plan **25** de l'image acquise.
La **figure 3** illustre l'architecture interne du dispositif de la **figure 2****.** Cette figure montre en perspective et en transparence les principaux éléments du dispositif. Le trou d'objectif **26** à mettre en regard de l'objectif de l'appareil électronique grand public; les moyens optiques, tels qu'au moins une lentille convergente **27,** sur leur support **28** pour les fixer mécaniquement dans l'alignement de l'axe optique **29** passant par le centre du trou d'objectif et le centre du plan de l'image à acquérir à la distance qui convient de l'objectif de l'appareil électronique grand public et du plan de l'image à acquérir. Des trous **30** sont prévus pour fixer, par exemple par des vis ou des rivets, une pièce intermédiaire d'adaptation à un modèle d'appareil électronique donné ou une pièce intermédiaire pourvue de moyens de réglage aptes à permettre une adaptation à une pluralité de modèles d'appareils électroniques grand public. Il est aussi prévu des variantes du dispositif qui sont directement compatibles avec des modèles d'appareils électroniques très largement diffusés sans avoir recours à une pièce intermédiaire d'adaptation. Le sous-ensemble électronique **31** est prévu pour comprendre la source de lumière **32,** ses moyens d'alimentation électrique autonomes, par exemple un accumulateur Li-ion polymère **33** rechargeable par une connexion USB **34** à un ordinateur ou à un chargeur comme pour la plupart des appareils électroniques grand public tels que les baladeurs, les téléphone portables ou les tablettes. Dans cette variante de mise en oeuvre évoluée, deux électrodes **35** déclenchent l'allumage de la source de lumière lorsqu'elles viennent au contact de la peau. Le contact avec une table ou tout support fait dans un matériau isolant ne provoquant pas l'allumage de la source de lumière. Une temporisation, qui éteint automatiquement la source de lumière lorsqu'elle arrive à échéance, est réinitialisée tant que les sondes sont au contact de la peau. Dans cet exemple, une roue porte-filtre **36** permet de choisir aisément le type de lumière par une rotation de la molette qui amène le filtre d'intérêt **37** devant la source de lumière, par exemple un filtre polarisant agencé pour permettre une mesure du relief cutané, un filtre polarisant agencé pour améliorer le contraste de l'image, un filtre laissant passer principalement la lumière ultraviolette issue d'une source produisant de la lumière blanche par excitation de phosphores. Il est prévu dans certaines variantes de réalisation, une position occultant partiellement la source de lumière, par exemple ne laissant sortir la lumière qu'en partie basse ou par une fente, de sorte à améliorer l'appréciation du relief cutané en produisant une lumière constituée de rayons parallèles. Une position **38** est prévue pour laisser passer intégralement la lumière de la source et un mécanisme d'indexation **39** permet un positionnement précis de chaque filtre devant la source. Cet exemple de mise en oeuvre comprend en outre une ouverture **40** et des moyens de guidage **41** pour recevoir des moyens optiques complémentaires **42** pour offrir un grossissement optique élevé de l'image, sur leur support **43.** Dans des variantes particulièrement élaborées du dispositif selon l'invention visant particulièrement les utilisations dans le domaine de la cosmétique, des moyens indirects électroniques sont prévus pour transformer l'expression d'au moins une caractéristique physique, chimique ou biologique de la peau de sorte à permettre une appréciation de l'au moins une caractéristique physique, chimique ou biologique de la peau par traitement d'image. Cette solution technique est particulièrement intéressante dans le cadre de dispositifs selon l'invention utilisables par des professionnels car les capteurs ne font pas appel à des solutions consommables et la précision des analyses est supérieure à celles que peuvent offrir les solutions à base de réactifs changeant de couleur. Cette dernière solution étant préférée lorsqu'un faible coût du dispositif est le critère principal de sélection des solutions techniques mises en oeuvre. Dans l'exemple non limitatif de la **figure 3****,** l'ajout de fonctions de transformation aux fonctions de base déjà décrites, comprend l'ajout d'un capteur d'humidité apte à fournir une mesure pouvant être linéarisée sur une large plage. Le capteur d'humidité est agencé pour venir en contact avec la peau de sorte à en mesurer l'humidité de surface lorsque le dispositif dans son ensemble est placé dans une position fonctionnelle. Il est prévu dans certaines variantes, de retirer les électrodes simples **35** prévues pour un fonctionnement en tout ou rien dans le cadre de la gestion automatique de la source de lumière, par un composant programmatique exécuté par un microcontrôleur. Ce programme est basé sur la détection du franchissement d'un seuil par le résultat de la mesure de l'humidité de surface pour piloter automatiquement la source de lumière. La source de lumière est activée automatiquement lorsque l'humidité de surface est supérieure ou égale à la valeur minimale de la peau humaine. La source de lumière est désactivée lorsque la valeur est inférieure au seuil pendant un temps préalablement déterminé, ce qui correspond à la pose sur une surface non organique lorsque le dispositif n'est pas utilisé. Il est prévu en outre d'ajouter des moyens pour évaluer l'élasticité de la peau. Des moyens basés sur l'amortissement d'impulsions vibratoires, sur le déplacement d'une fréquence de résonance, sur la propagation d'une onde vibratoire utilisant la peau comme milieu de propagation sont des solutions techniques particulièrement appropriées. Les moyens **44** sont agencés pour placer des moyens d'affichage dans le champ d'acquisition de l'image. Tout moyen d'affichage statique, c'est à dire non multiplexé, basé sur des technologies émissives telles que des diodes électroluminescentes LED ou OLED, est utilisable de même que des solutions basées sur le contrôle de la réflexion de la lumière ou sa transmission telles que des micro-miroirs, des LCD ou encore de l'encre électronique. Dans l'exemple de la **figure 3****,** des LED traditionnelles sont utilisées pour inclure l'information dans l'image. Très peu d'énergie est nécessaire pour que les LED actives **45** soient visibles lorsqu'elles sont orientées vers les moyens d'acquisition d'image. Avantageusement, les LED sont centrées dans des zones élémentaires matérialisées par des lignes noires et disposées en ligne ou en colonne pour une variable à transmettre donnée. Un codage de la valeur mesurée, par exemple en binaire permet de limiter le nombre de LED. Par exemple, 7 LED permettent de coder toutes les valeurs entières allant de 0 à 100. 5 LED sur une échelle où une seule LED est allumée à la fois permettent néanmoins de coder une variable sur une échelle de 0 à 100 par pas de 20. Les LED organisées en afficheurs à 7 segments est aussi une option prévue dans certaines variantes.
La **figure 4** illustre les moyens de fixation de l'exemple de la **figure 2****.** Cette figure illustre le cas de l'utilisation d'une pièce d'adaptation **46** prévue pour un modèle donné d'appareil électronique grand public **47.** Dans cette variante, la pièce d'adaptation est conçue pour un iPhone de la société Apple Computers (Marques déposées) pour fixer le dispositif selon l'invention **(46, 48)** par des clips pinçant l'appareil électronique **47** sur sa largeur et sur sa longueur. Ainsi les deux objets forment un ensemble solidaire et l'axe optique du dispositif **48** se trouve exactement aligné avec celui de l'objectif de l'appareil électronique **47** sans qu'il soit nécessaire de s'en préoccuper. Les clips sont en matière plastique élastique ou en métal flexible avantageusement revêtu d'un matériau qui assure la protection de la surface de l'appareil qui est à son contact.

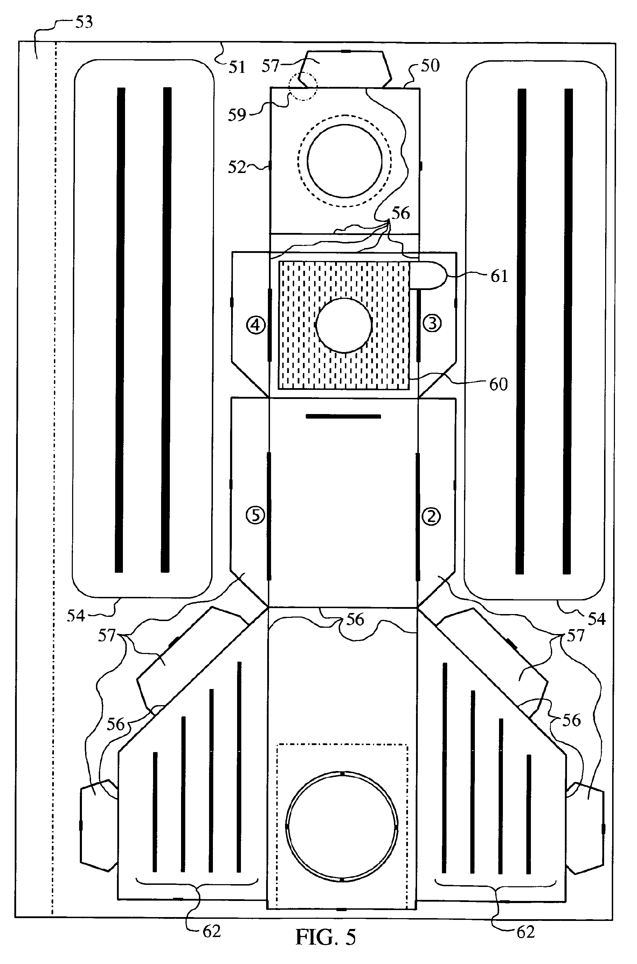

La **figure 5** illustre une sous-variante livrable à plat d'une première variante de moyens pour produire un grossissement optique de l'image. Il s'agit de la variante de mise en oeuvre préférée dans le cadre d'une application de l'invention dans le domaine de la cosmétique. Cette version du dispositif pour produire un grossissement optique de l'image est encartable dans un magazine ou peut être acheminée à son destinataire dans une enveloppe simple par les services postaux. Cette sous-variante est en outre peu coûteuse à fabriquer, ce qui la rend particulièrement apte à des opérations promotionnelles ou des campagnes massives de marketing. La fabrication requière seulement des opérations classiques d'impression, de découpe par emporte pièce, d'encollage et de pose de la lentille et d'un film de protection sur la surface adhésive. Toutes les étapes du procédé de fabrication de cette sous-variante sont en outre aisément automatisables et ne font appel qu'à des machines courantes aptes à produire de grandes quantités de dispositifs. Le montage final du dispositif est assuré par l'utilisateur. L'invention prévoit de faciliter le montage du dispositif par sa conception même. Ainsi, l'exemple de la **figure 5** prévoit la prédécoupe de la forme à plat du dispositif **50** dans une feuille de carton ou de matière plastique **51,** avantageusement choisie pour sa capacité à être recyclée ou biodégradée. La prédécoupe de la forme à plat du dispositif prévoit quelques attaches non découpées **52** réparties sur son périmètre pour la maintenir solidaire de la feuille 51 dans laquelle elle est incluse jusqu'à sa mise à disposition de l'utilisateur. Dans le cas d'un encartage du dispositif dans un magazine, une marge **53** est prévue du coté de la reliure. La **figure 5** représente la face externe du dispositif livré à plat. Les surfaces **54** de la feuille **51** qui ne comprennent pas d'éléments du dispositif, sont avantageusement utilisées pour recevoir des impressions relevant de la communication à des fins promotionnelle en relation avec la marque associée à l'opération de distribution du dispositif dans un contexte marchand ou en relation avec l'organisme à l'initiative d'une distribution du dispositif dans un contexte de dépistage du cancer de la peau. Dans cet exemple, le montage de la forme à plat **50,** après avoir été désolidarisée de son support 51, repose sur des pliages selon des lignes de pliures **56** qui sont avantageusement matérialisées par un amincissement de la matière selon une orientation facilitant le pliage dans la direction qui convient dans le but de rendre le montage intuitif. Le maintien du volume reconstitué après pliage repose sur des languettes **57** à introduire dans des fentes **58.** Tout ou partie desdites languettes étant agencées pour rester bloquées une fois insérées dans les fentes correspondantes, par leur forme **59** ou par l'usage de solutions à base d'adhésifs. La face du dispositif qui est prévue pour venir au contact de l'appareil électronique grand public est enduite d'un adhésif dit repositionnable, c'est-à-dire qui outre le fait de pouvoir être aisément décollé puis recollé à plusieurs reprises, garantit le respect de l'intégrité de la surface de l'appareil électronique grand public au contact duquel il se trouve. Il est aussi prévu des marquages sur une ou sur plusieurs des parties visibles du dispositif une fois monté pour apporter au moins une information à l'utilisateur. La **figure 5** illustre le marquage d'informations en rapport avec la destination des données produites par l'appareil électronique grand public. Ces informations **62** sont par exemple le numéro de téléphone d'un serveur apte à recevoir des MMS, une adresse de courrier électronique apte à recevoir des fichiers d'image en pièce jointe et une adresse de site web apte à permettre le chargement de fichiers d'images numériques dans la mémoire de masse d'un serveur distant.

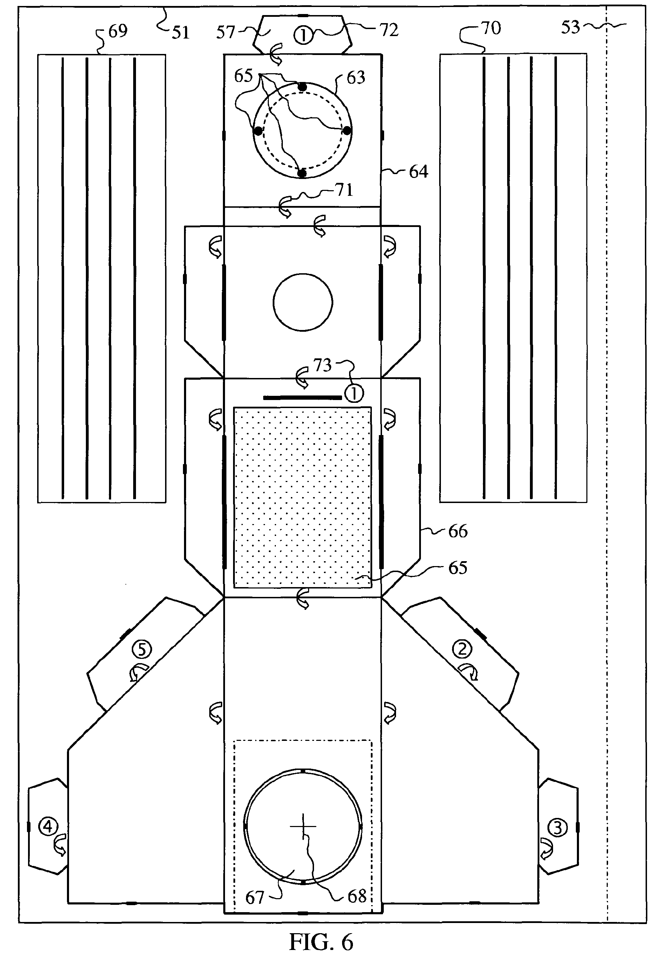

La **figure 6** illustre la face interne de la variante à plat de la **figure 5****.** Dans cet exemple de mise en oeuvre de l'invention, c'est vers l'intérieur que les différentes faces et languettes sont repliées. Il est prévu que les lignes de pliure soient marquées par l'outillage lors de la prédécoupe pour faciliter le pliage dans le sens qui convient. Les pré-pliures orientées se font au moyen d'une partie de l'outillage dont la section à la forme d'un V applique une pression sur la ligne de pliure suffisamment forte pour former la ligne de pliure mais sans aller jusqu'à couper la matière. La face interne reçoit en outre une lentille convergente **63** qui est fixée en alignement sur l'axe optique sur un étage intermédiaire **64** du dispositif. Ce plan intermédiaire est situé, après le montage final du dispositif, entre le plan qui est au contact de l'appareil électronique et celui qui est au contact de la peau. La fixation de la lentille est réalisée par exemple par des points de colles apposés sur ledit plan intermédiaire avant la pose de la lentille de sorte que la zone périphérique de la lentille recevant de la colle ne soit pas placée dans le champ de l'image. Un revêtement réfléchissant **65,** par exemple un film aluminisé, est apposé sur la le plan incliné **66** pour renvoyer la lumière arrivant par l'ouverture du dispositif dans le plan de l'image à acquérir. Dans certaines variantes de mise en oeuvre, le revêtement réfléchissant est amovible ou peut être masqué par un volet amovible complémentaire de sorte à éclairer le plan de l'image avec une lumière ayant des rayons inclinés selon un angle approprié pour mesurer le relief cutané dans des conditions d'éclairage optimales. Pour faciliter l'alignement de l'objectif de l'appareil électronique sur l'axe optique du dispositif il est prévu de mettre à profit la surface de matière **67** obturant la partie du plan de l'image à acquérir correspondant à la surface de peau et le cas échéant aux informations d'étalonnage. Une croix **68** ou tout autre élément graphique remarquable est imprimé à l'intersection du plan de l'image et de l'axe optique du dispositif et il est demandé à l'utilisateur de centrer cet élément dans l'image telle qu'elle apparait sur un écran de contrôle d'un appareil électronique. Lorsque la croix apparait bien au centre de l'image acquise par l'appareil électronique alors ce denier est posé sur la surface adhésive du dispositif pour solidariser les deux objets sans désaligner les axes optiques ni modifier l'orientation relative de l'appareil par rapport au dispositif. Il est prévu de faciliter le montage final du dispositif en utilisant une face du support **51** pour recevoir des impressions relatives aux instructions de montage **69** et aux instructions pour l'utilisation **70** du dispositif. Ces instructions pouvant avantageusement être réalisées sous la forme de bandes dessinées apte à être comprises dans toutes les langues. Il est prévu en outre des moyens pour guider l'utilisateur dans le montage final par des impressions sur des éléments du dispositif lui-même. Par exemple, des flèches **71** indiquant dans quelle direction faire les pliages, des numéros **72** sur les languettes **57** devant correspondre avec ceux des fentes **73** dans lesquelles elles doivent être insérées.

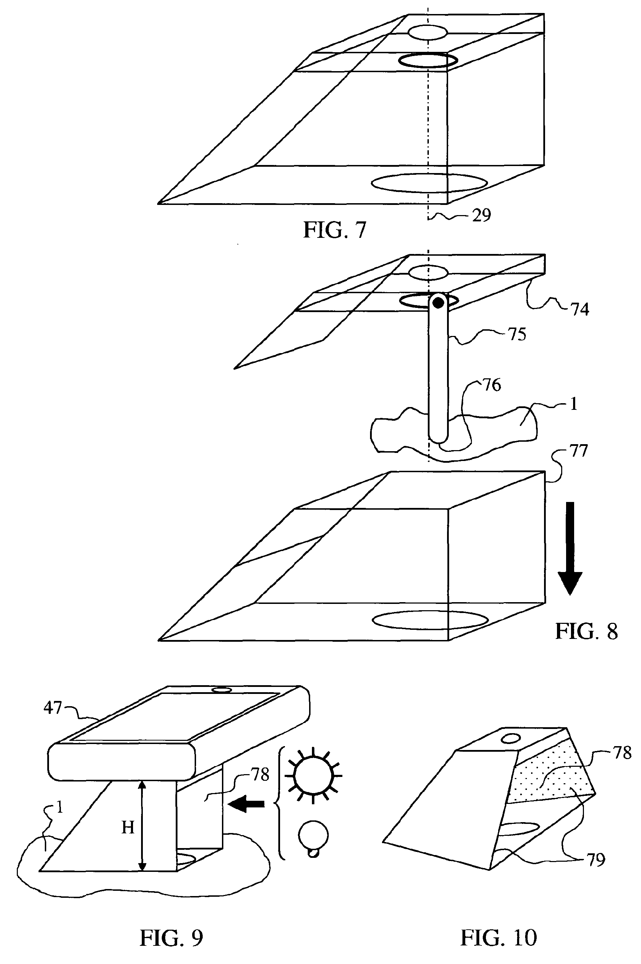

La **figure 7** illustre la variante à plat de la **figure 5** en présentée en volume. Le dispositif livré à plat donne l'objet en volume de la **figure 7** lorsqu'il est monté. Le centre du trou de la face venant en contact avec l'appareil électronique, le centre de la lentille et le centre de la face venant en contact avec la surface de peau se trouvent alignés sur l'axe optique **29.**
La **figure 8** illustre une sous-variante de la variante de la **figure 5****.** Cette sous-variante reprend le principe général du dispositif de la **figure 6** mais sous la forme de deux sous-ensembles désolidarisables. Cette sous-variante présente l'avantage de permettre l'acquisition d'images de surfaces de peau non planes telles que dans la région du contour des yeux, de la commissure des lèvres, du cou etc. Le premier sous-ensemble **74** comprend les moyens essentiels à l'acquisition d'image et des moyens pour maintenir l'ensemble formé par l'appareil électronique et ledit premier sous-ensemble du dispositif selon l'invention à la distance de la surface de peau qui convient pour que l'image soit nette. Les moyens pour maintenir à la bonne distance pour que l'image soit nette prennent la forme d'une béquille **75** dans l'exemple de la **figure 8****.** La distance déterminant la netteté de l'image est fixée par la mise en contact de l'extrémité de la béquille **76,** en position déployée si la béquille est escamotable, avec la peau **1.** L'extrémité de la béquille **76** étant agencée par sa forme et/ou par sa matière pour ne pas risquer de blesser la personne. L'optique de cette sous-variante est en outre agencée pour privilégier la profondeur de champ sur le grossissement de sorte à conserver une image nette malgré une variation significative de la distance des différents points sur les surfaces non planes d'intérêt. Il est prévu des versions ne comprenant que ledit premier sous-ensemble **74** du dispositif tel que présenté **figure 8****.** Dans ces versions compactes, la ou les béquilles d'appui sur la peau sont avantageusement escamotables ainsi que le cas échéant au moins une partie de la surface réfléchissante ou des moyens d'éclairage embarqués. D'autres versions comprennent les deux sous-ensembles **(74, 77),** le dispositif étant alors utilisable comme décrit dans les exemples des **figures 2** ou **5** lorsque les deux sous-ensembles sont assemblées. Ces versions offrent en outre la possibilité de désolidariser les deux sous-ensembles **(74, 77)** et de n'utiliser que le premier sous-ensemble **74** aux fins d'acquérir une ou plusieurs images de surfaces de peau à des endroits qui ne sont pas plans, ou dont la surface n'est pas suffisante pour pouvoir poser la base du second sous-ensemble **77,** ou pour lesquels la profondeur de champ est insuffisante pour que l'image soit nette lorsque la base du second sous-ensemble **77** est en appui sur des parties de corps protubérantes. Il est à noter que la partie réfléchissante solidaire du premier sous-ensemble peut être d'une surface réduite ou nulle par rapport à la surface qui est nécessaire pour éclairer convenablement la surface de peau dans une configuration où les deux sous-ensembles ont assemblés. En effet, utilisée seule, ladite première partie est suffisamment ajourée pour que suffisamment de lumière atteigne la surface de peau dont on souhaite acquérir l'image.
La **figure 9** illustre la variante à plat de la **figure 6** en situation. Le dispositif de la **figure 6** en perspective et en situation fonctionnelle, fixé à un appareil électronique grand public **47** et au contact d'une surface de peau **1.** La hauteur **H** du dispositif détermine par construction la distance entre l'objectif de l'appareil électronique **47** et la surface de peau **1** pour avoir une image nette. Une ouverture **78** à tourner vers une source de lumière naturelle ou artificielle externe éclaire le plan de l'image à acquérir.
**La** **figure 10** illustre une sous-variante de la variante à plat de la **figure 5****.** Cette sous-variante est agencée pour faire entrée davantage de lumière que dans la version de la **figure 5** par une ouverture **78** de plus grande surface. Les parois latérales **79,** qui sont recouvertes d'un revêtement réfléchissant comme la face réfléchissante opposée à l'ouverture, sont en outre utilisées pour contribuer à éclairer le plan de l'image à acquérir.

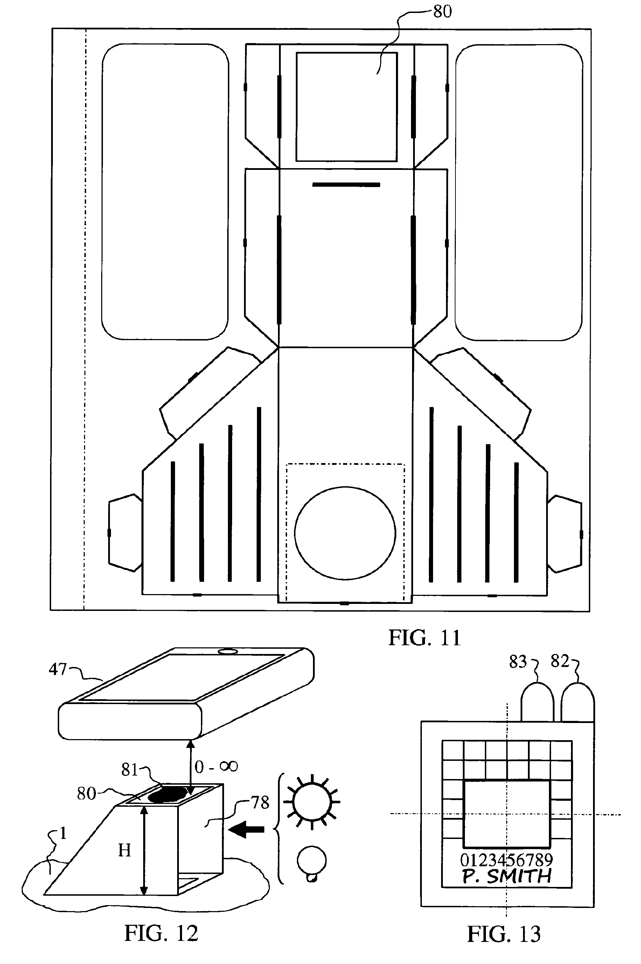

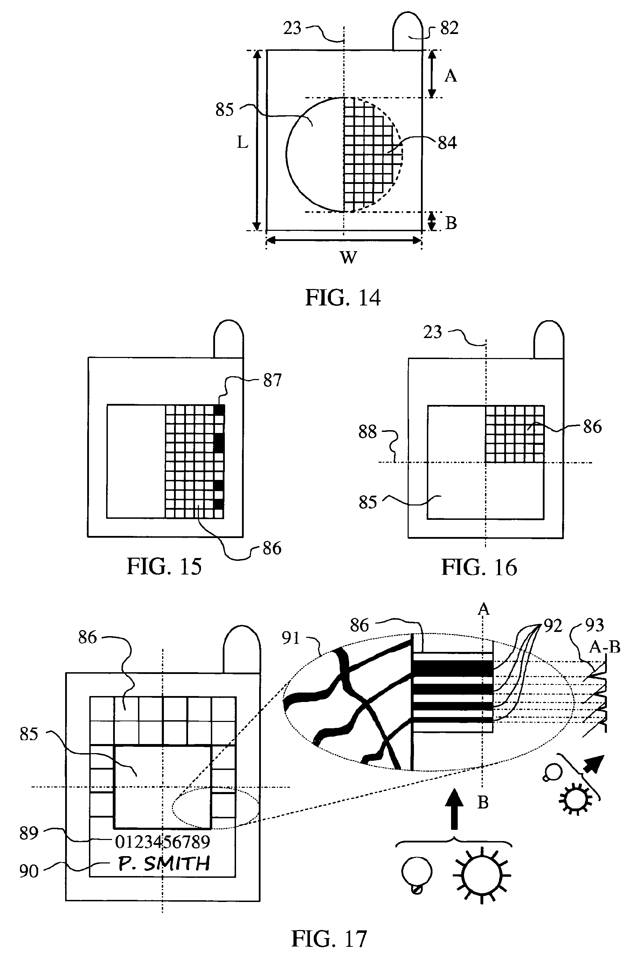

**La** **figure 11** illustre une seconde variante livrable à plat, face externe. Dans l'exemple de cette seconde variante, les moyens pour produire un grossissement optique de l'image comprennent une lentille de Fresnel unique de grande surface **80.** Dans cette variante, le plan de l'image à acquérir par l'appareil électronique n'est pas celui de la surface de peau mais celui de la surface de la lentille. Dans la pratique, à la distance naturelle de prise de vue compte tenu de la taille des objets, la profondeur de champ de la plupart des appareils est suffisante pour que l'image grossie et la surface de peau qui l'entoure soient toutes les deux nettes dans l'image acquise.
**La** **figure 12** illustre la variante à plat de la **figure 11** en situation. La lentille de Fresnel **80** est conçue pour rendre visible une image grossie **81** de la de surface de peau 1 à analyser placée à une distance H de la lentille. La distance H, fixée par construction lorsque la base du dispositif est au contact de la peau, détermine le grossissement. La distance H est déterminée pour maximiser le grossissement tout en garantissant une image nette et une profondeur de champ suffisante. La lentille, ou l'assemblage de lentilles le cas échéant, sont agencés pour que l'image grossie apparaissant dans le plan de la lentille soit nette indépendamment de la distance à laquelle se trouve l'objectif de l'appareil électronique grand public utilisé pour acquérir l'image.
**La** **figure 13** illustre des moyens consommables pour la seconde variante. L'utilisation d'une lentille de Fresnel offre de nombreux avantages dont celui de la légèreté. Le dispositif de la figure 11, réalisé intégralement dans des matériaux se présentant en feuilles, a une faible masse. Cette faible masse du dispositif rend possible son maintien temporaire en position fonctionnelle sur la peau, quelle que soit son orientation, par des moyens adhésifs. Il est ainsi prévu un sous-ensemble à usage unique ou limité dit consommable particulièrement adapté à cette variante du dispositif. Ce consommable, outre les fonctions d'étalonnage et de transformation selon l'invention et la présence d'un adhésif repositionnable déposé sur la face tournée vers la lentille pour assurer sa fixation sous la base du dispositif, comprend aussi un adhésif pour assurer sa fixation sur la peau. Un film de protection **82** à retirer avant utilisation est prévu sur la face du consommable du côté de la lentille et un autre film de protection **83** est prévu du côté à appliquer sur la peau.
**La** **figure 14** illustre des moyens pour étalonner la mesure ou l'évaluation des caractéristiques d'intérêt de l'image et/ou pour transformer selon l'invention des caractéristiques physiques, chimiques ou biologiques de la peau de sorte à pouvoir les apprécier par analyse d'image. Le but pour l'étalonnage est de s'affranchir de toutes les erreurs et les distorsions introduites dans la chaine d'acquisition et de transmission de l'image. La solution technique selon l'invention consiste à inclure dans le champ d'acquisition de l'image des éléments dont au moins une caractéristique est connue. Ainsi les logiciels de traitement d'image appropriés disposent d'une référence dans l'image qui leur permet de corriger cette dernière de sorte à retrouver la valeur connue de la caractéristique pour l'élément de référence. La correction donnant le meilleur résultat pour l'élément de référence est ensuite appliquée au reste de l'image. Des raffinements sont prévus pour améliorer la précision des corrections en tenant compte de la localisation des pixels grâce à la connaissance de caractéristiques optiques des moyens pour rendre l'image nette selon l'invention. L'exemple présenté illustre l'utilisation de moyens dits consommables pour recevoir les éléments d'étalonnage et, le cas échéant, des éléments de conversion dans la zone **84.** La présence d'une grille régulière dans la zone **84** est particulièrement avantageuse. La grille permet de délimiter avec un fort contraste qui facilite les traitements d'image ultérieurs, chaque zone portant des couleurs et/ou des surfaces ayant un éclat, c'est-à-dire un niveau de réflexion de la lumière, déterminés et/ou des réactifs changeant de couleur sous l'action d'une caractéristique physique, chimique ou biologique de la peau. La grille permet en outre d'évaluer les distorsions géométriques en type et en intensité introduites par les composants optiques et de les corriger à posteriori par des traitements d'image appropriés. La grille permet en outre de corriger plus efficacement les aberrations chromatiques par l'exécution de traitements d'image appropriés en ce que la grille apparaissant en noir donne la position exacte des pixels pour lesquels des déplacements sont observés pour certaines composantes de leur couleur et que par conséquent les logiciels appropriés opèrent les décalages inverses et reconstituent les couleurs réelles des pixels de l'ensemble de l'image. Par ailleurs, la grille marque, de la manière la plus contrastée, les limites des zones comprenant des éléments d'étalonnage et/ou de conversion, ceci améliore l'efficacité des logiciels de traitement qui centrent l'échantillonnage des pixels au centre des cases élémentaires concernées pour éliminer tout risque de perturbations aux frontières de chaque zone. En outre une grille est un motif facilement reconstituable par logiciel en cas de pixels manquants par la connaissance de ses dimensions et de son pas qui seront avantageusement constants. La présence dans l'image d'une grille d'au moins une case élémentaire, ayant des dimensions connues en abscisse et en ordonnée, ainsi qu'ayant si possible une largeur de trait maitrisée et connue, offre en outre l'avantage de permettre la mesure précise des éléments de l'image par traitement d'image ultérieur sans nécessiter une grande précision absolue dans la maîtrise des caractéristiques dimensionnelles et optiques des moyens mis en oeuvre dans la chaine d'acquisition des images. La connaissance des dimensions réelles des éléments de l'image permet en outre de remettre une pluralité d'images à la même échelle en vu de leur comparaison. La comparaison des images, après d'éventuels traitements complémentaires de translation et/ou de rotation de l'ensemble des pixels de l'image pour ramener un élément d'intérêt au même endroit de l'image préparée en vue de la comparaison et dans une position identique pour toutes les images, se fait avantageusement par l'exécution de logiciels de comparaison automatique des images d'un même objet d'intérêt tel qu'un grain de beauté ou une lésion. Il est prévu en outre que la comparaison des images puisse se faire par l'oeil humain par affichage simultané des images sur un écran, en juxtaposition ou en superposition, notamment aux fins de contrôler a posteriori la raison d'une alerte ayant été généré automatiquement par les moyens de comparaison basés sur des logiciels de traitement d'images. La présence d'éléments de référence dans chaque image permet en outre de faire bénéficier les images anciennes de progrès réalisés dans les traitements d'image en exécutant les nouveaux traitements d'image sur les images anciennes en vue d'analyser de nouvelles caractéristiques de la peau ou de produire des mesures plus précises de caractéristiques précédemment analysées. La zone **85** est percée pour faire apparaître la surface de peau dans le champ d'acquisition de l'image. Cet exemple exploite la symétrie selon l'axe principal **23** comme expliqué à la **figure 2****.** Il est prévu que les moyens consommables de la **figure 14** se fixent sur la base d'un dispositif tels que ceux des **figures 2** ou **5** par un adhésif repositionnable protégé par un film amovible **82** jusqu'à son utilisation. Un détrompage est prévu pour empêcher l'utilisateur de se tromper d'orientation lors de la pose du consommable. Une seule position est possible sachant que l'adhésif est présent sur une seule face dans cette variante et que le positionnement de la fenêtre est asymétrique (la longueur L est différente de la largeur W et les grandeurs A et B sont différentes).
La **figure 15** illustre une autre disposition des moyens consommables de la **figure 14****.** Dans cet exemple la fenêtre d'analyse, d'étalonnage et le cas échéant de conversion est rectangulaire au lieu d'être ronde, ce qui maximise la surface d'image utilisable aux fins de l'étalonnage et/ou de la conversion. Suffisamment de zones élémentaires **86** pour l'étalonnage étant présentes dans l'image, il devient possible d'utiliser des zones élémentaires pour coder le type de consommable et connaitre toutes ses caractéristiques par l'intermédiaire d'une base de données. Dans cet exemple, les zones élémentaires de la colonne **87,** selon qu'elles sont blanches ou noires, codent la référence du consommable en binaire.
La **figure 16** illustre l'utilisation de deux axes de symétrie. Cet exemple met à profit l'existence d'un second axe de symétrie **88** qui est perpendiculaire à l'axe de symétrie principal **23** et qui le coupe au centre de l'image. Le second axe de symétrie coupe aussi ainsi l'axe optique du dispositif selon l'invention qui est aligné sur celui de l'appareil électronique. L'exploitation du second axe de symétrie est prévue pour augmenter la surface relative dans l'image de la zone percée **85** découvrant la peau. Dans cette variante, il est prévu de tenir compte du fait que le second axe de symétrie **88** est un axe de symétrie pour les moyens optiques mais pas pour la source de lumière. Ainsi, il est prévu que l'affectation des zones élémentaires **86** tienne compte de cette différence pour obtenir les meilleurs résultats. Par exemple les zones élémentaires affectées à l'étalonnage du rendu des couleurs, dont le rendu ne dépend pas de la luminosité seront de préférence éloignées du centre de l'image pour pouvoir en rapprocher d'autres, dont le rendu peut être influencé par des variations du niveau d'éclairement, des zones d'éclairement les plus homogènes.
La **figure 17** illustre une variante préférée de consommable qui offre l'avantage d'affecter la zone centrale de l'image à la partie percée **85** découvrant la peau. Cet exemple suppose l'utilisation de moyens optiques dont les caractéristiques sont connues, caractérisées et reproductibles. Dans le cas de l'utilisation de moyens optiques introduisant des défauts dans l'image nécessitant une correction, les caractéristiques desdits moyens optiques sont identifiés par un moyen d'identification inclus dans l'image au niveau du dispositif lui-même (non représenté sur la **figure 17****).** Dans le cas de l'utilisation de moyens optiques n'introduisant pas de défauts notables dans l'image ou introduisant des défauts connus, dûment répertoriés, ces moyens optiques étant identifiables de sorte à pouvoir corriger leurs défauts sans avoir recours à des moyens d'étalonnages présents dans l'image. Les défauts aisément corrigeables de cette façon sont les distorsions géométriques et les aberrations de tous types dont le vignettage. Il ne reste plus que le besoin d'étalonner le rendu des couleurs et la sensibilité de la chaine d'acquisition d'image. Le cas échéant, des moyens pour convertir des caractéristiques physiques, chimiques ou biologique en variation de couleur d'une ou de plusieurs zones élémentaires peuvent nécessiter des éléments d'étalonnage ou des témoins qui leurs soient spécifiques. Dans les conditions de cet exemple, moins de zones élémentaires **86** sont nécessaires à la mise en oeuvre de l'invention et ces zones sont distribuées autour de la zone centrale percée **85** découvrant la peau.
Il est prévu qu'une diminution du nombre de zones élémentaires **86** par rapport aux autres variantes précédentes soit compensée par une augmentation du nombre de références de consommables pour pouvoir répondre à l'ensemble des besoins. Les caractéristiques des moyens consommables sont identifiés par un moyen d'identification **89** inclus dans l'image au niveau du consommable lui-même ce qui permet d'exécuter les traitements d'images appropriés. Généralement les moyens de traitement exécutent une requête dans une base de données à partir du code **89** du consommable, la base renvoyant les caractéristiques détaillées du consommable et le cas échéant des informations en relation avec les traitements correspondant. Ces informations étant par exemple le nom du programme de traitement à exécuter accompagné des paramètres appropriés ou un composant programmatique complet prêt à être exécuté. Cet exemple de consommable comprend en outre une zone **90** permettant à l'utilisateur d'entrer au moins un élément d'information dans l'image. Il s'agit dans cet exemple d'une zone pour écrire librement par exemple un nom, d'autres variantes sont prévues pour faire des choix dans des listes fermées de propositions. Le détail agrandi **91** illustre plus particulièrement les moyens qui sont avantageusement mis en oeuvre pour apprécier le relief cutané selon l'invention, c'est-à-dire par des moyens relevant principalement de l'optique et des traitements d'image. Au moins une zone élémentaire **86** comprend des reliefs **92** ayant des hauteurs connues, lesdits reliefs étant agencés par ordre de hauteurs croissantes, la plus petite hauteur étant la plus proche de la source de lumière comme le montre la coupe A-B. Les traitements d'images pour mesurer le relief cutané mesurent la largeur de l'ombre portée sous un éclairage constitué principalement de rayons parallèles inclinés sous un angle approprié **93.** La mesure du relief cutané se fait après une étape d'étalonnage qui prend pour référence l'au moins une zone élémentaire comprenant des éléments de relief ayant des hauteurs connues. En pratique, un compromis doit être trouvé entre la pureté de la lumière éclairant l'image à acquérir relativement aux rayons parallèles d'intérêt et la quantité de lumière entrant pour obtenir des ombres portées ayant un contraste suffisant pour être exploitées par les traitements d'image.

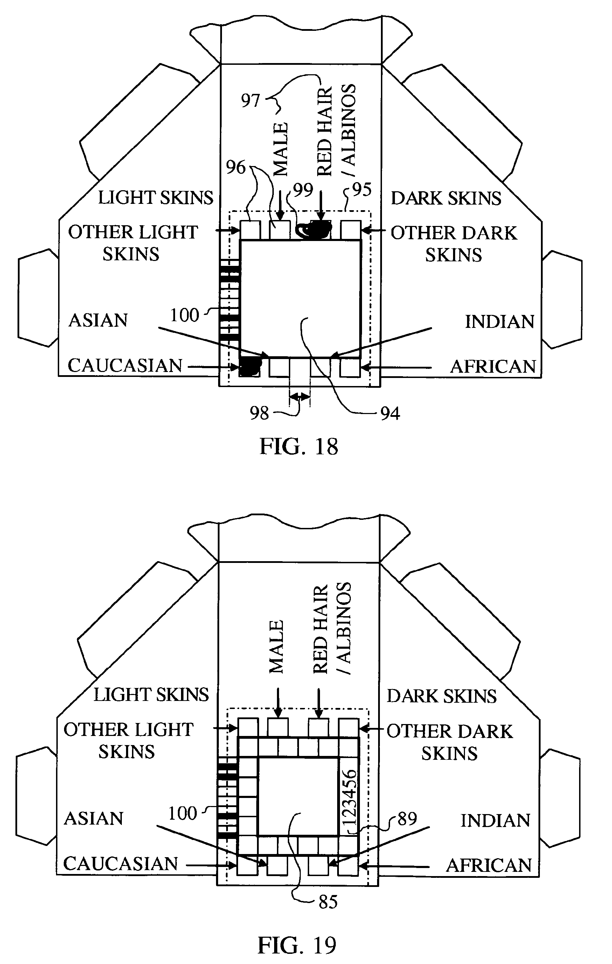

La **figure 18** illustre des moyens pour entrer des d'informations dans l'image. Cette figure illustre un exemple de moyens pour permettre à l'utilisateur d'entrer au moins un élément d'information dans l'image dans le cas de la variante du dispositif de la **figure 5****.** Ceci est particulièrement avantageux car le dispositif de la **figure 5** qui est livré à plat et qui comprend déjà des impressions à d'autres fins, le surcoût pour l'ajout de ces moyens d'entrée d'information est nul. Il est prévu que la zone percée **94** dans la base du dispositif (à ne pas confondre avec la zone percée **85** dans un consommable puisse avoir une forme quelconque, par exemple ronde **figure 5****,** rectangulaire dans l'exemple de la **figure 18****.** La zone découpée **94** est comprise dans le cadre **95** délimitant l'image à acquérir. Il est prévu que l'espace compris entre la zone percée **94** et le cadre **95** délimitant l'image à acquérir, soit occupé par des zones délimitées **96** qui sont associées à des choix exprimés en langage naturel **97** ou par référence dans une légende. Il est prévu que les zones à noircir pour exprimer un choix soient séparées les unes des autres par un espace **98** pour absorber d'éventuels débordements **99** lors du noircissement d'une zone **96** et éviter qu'il soit interprété par les traitements d'image comme le noircissement d'une zone adjacente. Il est aussi prévu dans certaines variantes de sélectionner une proposition en entourant les propositions retenues d'un trait et/ou en rayant les propositions rejetées. Il est également prévu des moyens pour identifier chaque variante de moyens pour entrer des informations par choix dans des listes fermées. Il peut s'agir par exemple d'un code à barres 1D **100** comme dans cet exemple ou d'un code dit 2D plus compact, de l'utilisation directe de caractères alphanumériques comme dans la **figure 17****,** de codage en binaire comme dans la **figure 15** ou de tout autre moyen pour encoder une référence dans une image qui puisse faire l'objet d'une reconnaissance et d'un décodage par un traitement approprié de l'image. Il est à noter que les programmes informatiques élémentaires permettant de reconnaitre dans une image, la valeur d'un code à barres 1D ou 2D, une suite de caractères alphanumériques et de nombreux autres moyens de codage exploitables dans le cadre de l'invention aux fins d'identifier les variantes de dispositifs ou de consommables sont compris dans l'état de la technique. Dans cet exemple illustrant le cas d'un dispositif à très faible coût, il est prévu qu'il soit à usage unique ou pour un usage occasionnel et dans la plupart des cas pour un utilisateur unique. Dans ce contexte, il est particulièrement pertinent d'utiliser la surface imprimable de la face intérieure du dispositif, sur laquelle il est possible d'écrire pour entrer des informations avant son montage final. Dans cet exemple, l'entrée des informations par l'utilisateur décrivant indirectement les caractéristiques principales de sa peau en précisant l'ethnie à laquelle il appartient, est particulièrement utile. La connaissance de l'ethnie permet par exemple d'affiner les traitements d'image et aussi d'obtenir des informations complémentaires contenues dans des bases de données qui associent des caractéristiques de la peau avec des caractères génétiques en relation avec le type ethnique des personnes. Dans cet exemple, les traitements d'image, qui connaissent les choix proposés et leur localisation après la lecture du code **100** et l'interrogation de la base de données correspondante, décodent que l'utilisateur est une femme de type caucasien ayant une peau de rousse.
La **figure 19** illustre les moyens de la **figure 18** sous la base desquels un consommable approprié a été collé pour assurer l'étalonnage des traitements d'image et/ou la conversion de paramètres physiques, chimiques ou biologiques. Dans cet exemple, les moyens pour entrer au moins une information sont identifiés par un code **100** et le consommable est identifié par un code **89.** La surface de peau à analyser apparait dans la zone percée **85** du consommable. Bien entendu on ne sort pas du cadre de l'invention si les moyens d'étalonnage et/ou de conversion sont directement déposés sur la face interne du dispositif au lieu de l'être sur des moyens remplaçables dits consommables.

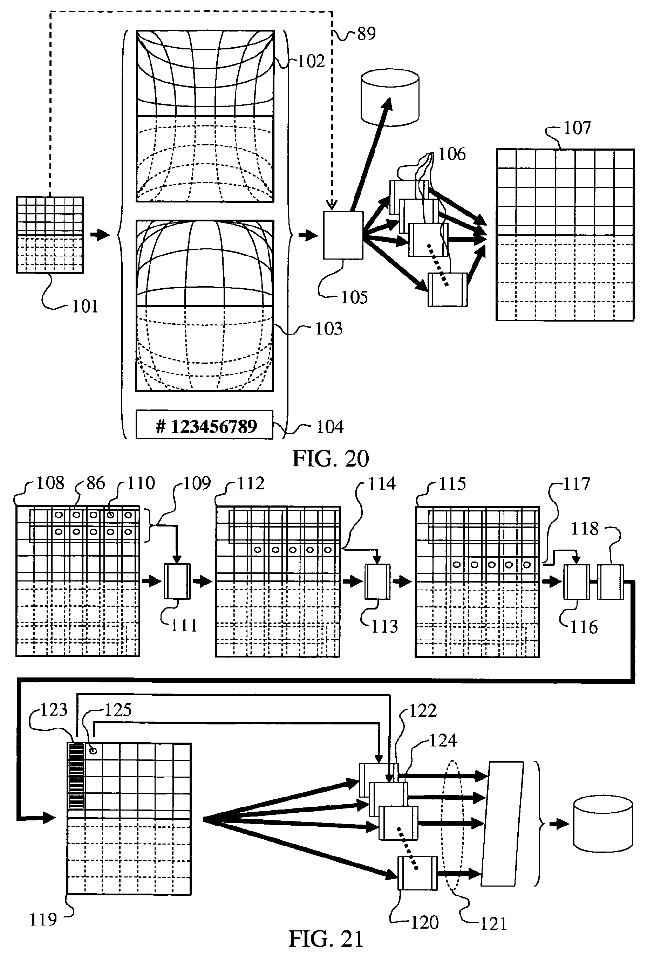

La **figure 20** illustre des traitements d'image pour corriger des distorsions géométriques dans le cas de l'utilisation de moyens optiques à faible coût. Une optique simplifiée à une seule lentille, en particulier s'il s'agit d'une lentille dite de Fresnel, fabriquée avec des matériaux et par des procédés à faible coût est susceptible d'introduire des distorsions géométriques. Par exemple il peut s'agir de distorsions dites en coussinet ou en barillet qui déforment l'image d'une grille régulière **101** respectivement comme dans les représentations **102** ou **103.** Dans certaines variantes, il est prévu avantageusement de rechercher non pas une optique qui ne déforme pas l'image mais de fabriquer de manière reproductible une optique qui déforme l'image d'une manière connue. La correction se faisant alors par application d'un profil de traitement approprié appelé en fonction d'un identifiant de l'optique **104.** Il est également prévu une autre approche qui repose sur des traitements d'image qui sont aptes à corriger tout ou partie des distorsions et des aberrations sans qu'il leur soit nécessaire de connaitre des caractéristiques de l'optique ni possiblement celles de la grille **89.** Chaque approche ayant ses avantages et ses inconvénients. La connaissance de caractéristiques de l'optique permettant en général de simplifier les programmes de traitement d'image et de diminuer les temps d'exécution au prix de la gestion d'informations supplémentaires. Il est prévu d'arbitrer entre les différentes options précédentes en fonction des contraintes matérielles et économiques lors de la mise en oeuvre de l'invention. Dans cet exemple non limitatif, un premier traitement **105** stocke et indexe l'image brute acquise dans une base de données puis appelle le ou les traitements d'image élémentaires **106** correspondant aux distorsions à corriger pour produire des données codant une image résultante **107** dans laquelle les distorsions géométriques ont été corrigées. Il est possible de mettre en oeuvre l'invention en stockant les images corrigées plutôt que les images brutes, cela étant, il sera généralement préféré lorsque la puissance de traitement informatique n'est pas considérée comme une ressource rare, de stocker les images brutes pour ne pas perdre d'information et pouvoir tirer parti des améliorations continues des programmes informatiques de traitement d'image. Il est ainsi préféré de traiter à nouveau à chaque nouveau besoin des images anciennes avec les dernières versions des programmes de traitement d'image disponibles.

La **figure 21** illustre des traitements d'image pour étalonner des caractéristiques de l'image, pour corriger des aberrations chromatiques, pour évaluer des caractéristiques de la peau par l'intermédiaire de caractéristiques de l'image qui varient en fonction des caractéristiques de la peau.
Cet exemple de mise en oeuvre du procédé selon l'invention illustre des traitements d'image exécutés en séquence ou en parallèle selon les cas. La première image **108** dans cette chaine de traitements est avantageusement celle qui est issue d'une étape préalable de correction des distorsions géométriques (avantageusement l'image **108** est l'image **107** de la **figure 20****).** Une étape visant à étalonner le rendu des couleurs utilise les références de couleur des deux premières lignes **109.** Pour éviter de subir l'effet des possibles aberrations chromatiques non encore corrigées, les références de couleurs prises en compte sont celles de groupes de pixels **110** éloignés des bords des zones élémentaires **86.** Outre les trois couleurs fondamentales pures, le jaune, le magenta et le cyan plus un noir et un blanc, il sera avantageusement ajouté un nuancier typique de couleurs de peau compte tenu de la gamme de couleur relativement restreinte concernée et de la délicatesse des nuances à discriminer au sein de cette gamme. L'exécution du traitement **111** produit une image intermédiaire **112** dont la couleur de chaque pixel a été corrigée en fonction du rendu initial des couleurs de référence **109.**
Le traitement suivant **113** vise la correction du vignettage et/ou de l'effet d'une distribution non homogène de la lumière sur la surface de l'image à acquérir. La correction repose sur des références de couleur et de brillance identiques, par exemple un blanc satiné correspondant au milieu d'une échelle de brillance. Les zones élémentaires recevant la couleur de référence pour ce traitement d'image seront judicieusement réparties en fonction des caractéristiques des moyens d'éclairage. Par exemple continument sur toutes les zones élémentaires d'une ligne **114** parallèle à l'axe de symétrie de la source de lumière et pas trop éloigné du centre de l'image. A l'issue du traitement **113** est produite une image intermédiaire **115** dans laquelle la luminosité des pixels a été corrigée en fonction du rendu initial des références identiques en fonction de leur localisation et en tenant compte le cas échéant des lois de répartition de la lumière des moyens optiques utilisés pour les pixels éloignés des zones de référence et de leur symétriques relativement à au moins un axe de symétrie.
Le traitement suivant **116** vise à étalonner le rendu de la brillance par rapport à la gamme de brillance que l'on peut rencontrer en matière de peau humaine pour pouvoir exploiter au mieux la dynamique de la chaine d'acquisition de l'image selon ce critère. La correction repose sur des références **117** comprenant par exemple quelques zones élémentaires, chacune ayant un niveau de brillance différent mais réaliste, et la même couleur telle qu'un beige correspondant à une peau de tonalité moyenne. Il sera avantageusement ajouté une zone d'une couleur correspondant à une peau noire mate réaliste et une zone d'une couleur correspondant à une peau claire et grasse réaliste, ceci pour étalonner les valeurs extrêmes. Un traitement **118** de correction des aberrations chromatiques est avantageusement appliqué à l'image issue du traitement **116** pour produire une image **119** corrigée et étalonnées apte à l'exécution de traitements **120** visant l'analyse de caractéristiques de la peau proprement dite c'est-à-dire des traitements visant la production de données **121** quantitatives et/ou d'indications qualitatives en relation avec des caractéristiques de la peau.
Les caractéristiques d'intérêt pour lesquelles des traitements d'image appropriés sont exécutés dépendent du domaine d'application de l'invention. Par exemple dans le domaine de la cosmétique, il est souhaitable de caractériser la peau analysée sur les plans de :
- La couleur,
- La brillance,
- L'humidité en surface,
- La texture,
- Le relief cutané,
- La pigmentation,
- L'élasticité,
- La présence de rides,
- La présence de kératine,
- La présence d'impuretés,
- La présence de pores visibles ou de lésions,
- La taille le nombre de pores visibles par unité de surface,
- L'acidité de surface,
- La sensibilité à certains produits,
- Le type de flore microbienne colonisant la surface etc.

Dans l'exemple de la **figure 21****,** un traitement **122** s'appuyant sur les informations d'étalonnage de la colonne **123** vise à quantifier le relief cutané. Un traitement d'image **124** s'appuyant sur la couleur de la zone élémentaire **125** qui correspond à l'image vue par transparence d'un réactif qui est imprimé sur la face du consommable venant en contact avec la peau lors de l'acquisition de l'image, ledit consommable étant constitué d'une feuille en matière plastique transparente sur laquelle les différentes couleurs et réactifs sont déposés par exemple en utilisant des procédés classiques de tampographie ou de sérigraphie sur une face et/ou sur l'autre selon leur fonction. Ce réactif vire du bleu intense vers le rose vif en fonction du pourcentage d'humidité à la surface de la peau. Il est prévu d'appliquer cette même solution technique avec des réactifs différents placés dans d'autres zones élémentaires du consommable pour évaluer d'autres caractéristiques physiques, chimiques ou biologiques de la peau.
Les données **121** produites par les moyens et procédés selon l'invention sont alors aptes à être stockées et/ou exploitées par tout procédé d'ordre supérieur.

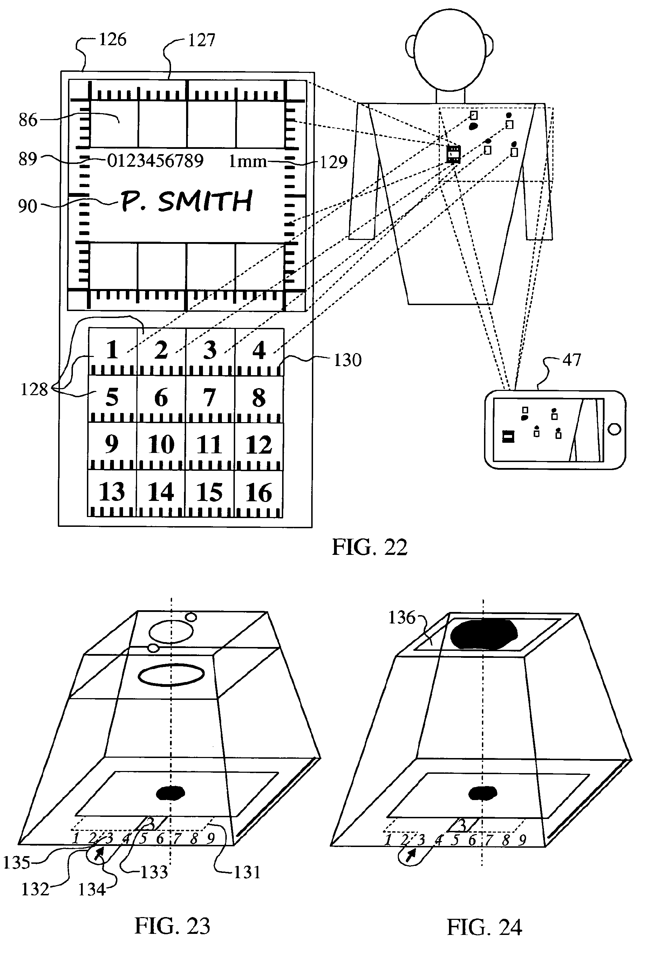

La **figure 22** illustre une utilisation de l'invention dans le domaine de la détection du cancer de la peau.
Un premier problème consiste à inventorier et à répertorier les grains de beauté et les lésions à surveiller, sachant qu'en général une personne en a plusieurs répartis sur des surfaces de peau pouvant être relativement grandes, par exemple sur tout le haut du dos. Il est prévu un consommable **126** sous la forme d'une feuille comprenant plusieurs éléments autocollants **(127, 128)** utilisant un adhésif de force d'adhésion moyenne, plusieurs fois repositionnable et apte à être en contact avec la peau. Un premier élément **127** du consommable de la **figure 22** est un moyen pour introduire dans l'image à acquérir des références dimensionnelles en abscisse et en ordonnée. L'unité de graduation est par exemple le millimètre et elle est avantageusement indiquée explicitement **129.** Avantageusement une zone **90** est aménagée pour permettre l'entrée d'au moins une information par l'utilisateur telle qu'un nom, une date. Un code d'identification **89** du consommable ainsi que des zones élémentaire d'étalonnage et/ou de conversion **86** sont prévues dans certaines variantes. Il est prévu que le consommable comprenne en outre une pluralité d'étiquettes autocollantes numérotées **128.** Ces étiquettes **128** comprennent avantageusement aussi des références dimensionnelles **130** dans une ou dans deux dimensions. L'invention exploite dans cette variante l'aptitude native des appareils électroniques grand public **47** à acquérir des images d'une large surface de peau pour référencer chaque grain de beauté et/ou lésion à surveiller par un numéro au moyen d'étiquettes autocollantes numérotées **128** faisant partie de moyens consommables **126** prévus pour la mise en oeuvre de l'invention. L'image ou les images numériques d'ensemble résultantes sont mémorisées dans des moyens de stockage de données, locaux ou distants, et/ou imprimées pour garder en mémoire le numéro de chaque grain de beauté ou de chaque lésion pour pouvoir suivre son évolution au cours du temps.
Par ailleurs, certains modèles d'appareils électroniques particulièrement performants, ayant une résolution élevée et une capacité de mise au point rapprochée de type macro photo, permettent la mise en oeuvre de l'invention sans nécessiter de moyens complémentaires pour produire un grossissement optique et pour rendre l'image nette à une plus courte distance que celle permise par les moyens optiques natifs de l'appareil.
La **figure 23** illustre un raffinement fonctionnel de ladite première variante du dispositif selon l'invention. Les moyens pour produire un grossissement optique et pour rendre l'image nette à une plus courte distance que celle permise par les moyens optiques natifs de l'appareil, sont avantageusement complétés par des moyens pour inclure dans l'image le numéro associé de manière pérenne dans le temps au grain de beauté ou à la lésion dont on souhaite acquérir une image rapprochée et grossie. La **figure 23** illustre un exemple de solution mécanique basée sur une réglette **131** placée sous la base du dispositif et déplaçable en translation par l'utilisateur au moyen d'une languette **132.** Une fenêtre **133** dévoile le numéro choisi dans le champ de l'image. Avantageusement, un index **134** solidaire de la languette **132** et qui pointe sur un numéro **135** imprimé sur une face externe du dispositif, indique clairement à l'utilisateur le numéro **133** qui apparait dans la fenêtre à l'intérieur du dispositif. Ainsi, l'utilisateur peut acquérir régulièrement des images précises de chaque objet d'intérêt sur sa peau sans difficulté ni risque d'erreur sur l'objet par l'inclusion du numéro **133** dans l'image. A chaque nouvelle acquisition d'image, l'utilisateur rappelle la ou les images d'ensemble préalablement acquises pour prendre connaissance à nouveau de manière certaine du numéro affecté à chaque objet d'intérêt. Le système selon l'invention, qui pour un utilisateur donné garde avantageusement en mémoire la ou les images d'ensemble préalablement acquises, peut lui fournir à sa demande quand il le souhaite cette ou ces images d'ensemble par l'intermédiaire d'un moyen électronique d'affichage ou d'impression dont l'utilisateur dispose. En outre, il est prévu dans le système selon l'invention, des traitements pour indexer automatiquement les images objet par objet en fonction de leur numéro et de les associer à l'image ou aux images dites d'ensemble.
La **figure 24** illustre le raffinement fonctionnel de la **figure 23** transposé dans le contexte de ladite seconde variante du dispositif selon l'invention qui comprend des moyens **136** pour produire un grossissement optique et pour rendre l'image nette indépendamment de la distance à laquelle se trouve l'objectif de l'appareil électronique grand public utilisé pour acquérir l'image.

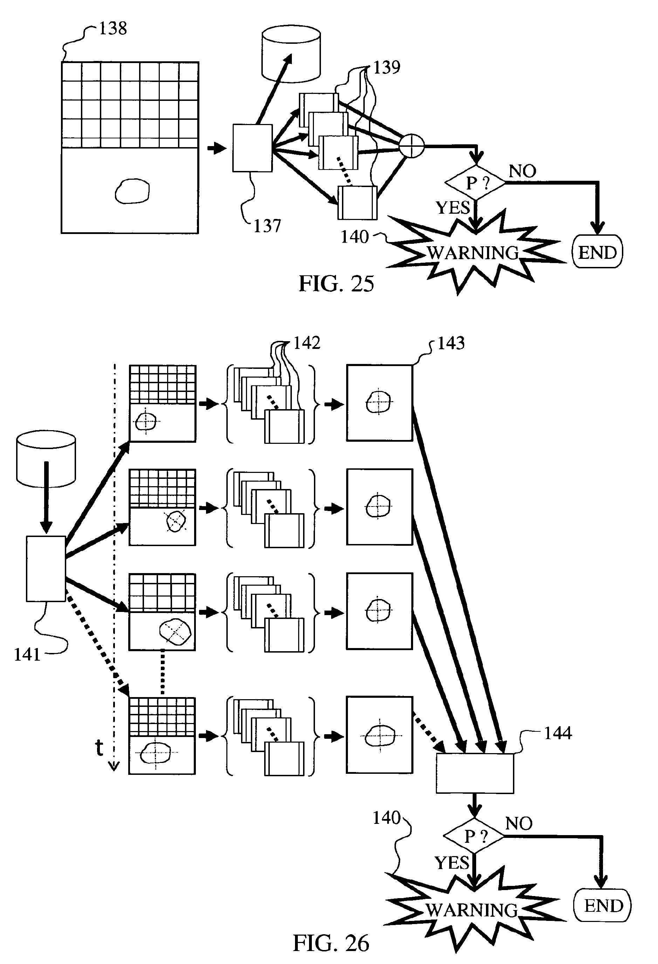

La **figure 25** illustre la détection de problèmes par traitements d'image sur des images isolées. Dans le domaine de la détection précoce du cancer de la peau, il est prévu d'exécuter des traitements sur des images de grains de beauté ou de lésions pour détecter les indices suivants qui sont reconnus pertinents par les dermatologues :
- De la couleur noire apparaissant dans la lésion,
- Une forme qui devient asymétrique,
- Des bords irréguliers,
- Une coloration non homogène,
- Un grand diamètre (supérieur à 6 mm),
- Une évolution dans le temps de la lésion.

On distingue en matière de traitement d'image selon l'invention un premier groupe de traitements d'image dits sur image isolée qui sont exécutés image par image et un second groupe de traitements dits sur images multiples qui sont exécutés sur une pluralité d'images des mêmes objets d'intérêt acquises à des instants différents. Naturellement, il est prévu que toutes les images sur lesquelles sont appliqués des traitements sur images multiples ont fait l'objet préalablement de traitements d'image sur image isolée.
Des traitements préalables **137** tels qu'un stockage dans une base de données, des traitements de correction et/ou d'étalonnage et/ou de transformation selon l'invention tels que décrits précédemment sont appliqués à une image **138.** L'image résultante subit une batterie de traitements spécialisés **139** dans le but de rechercher au moins les cinq premiers indices de la liste précédente. Il est prévu qu'un problème soit détecté **140** et signalé à un procédé d'ordre supérieur si au moins un indice pertinent a été détecté à l'issue du traitement correspondant.
La **figure 26** illustre la détection de problèmes par traitements d'image dits sur images multiples. Des images préalablement acquises subissent le cas échéant des traitements **141** de correction et/ou d'étalonnage et/ou de transformation selon l'invention tels que décrits précédemment. Les images sont en outre ordonnées en fonction de l'horodatage de leur acquisition au moyen par exemple d'informations associées au système de fichiers ou contenues dans les métadonnées associées à chaque image numérique. Les images résultantes sont ensuite l'objet de traitements **142** visant à les normaliser préalablement à l'application de traitements de comparaison. On entend par normaliser les images, appliquer aux pixels de ces images des traitements tels que des translations et/ou des rotations visant à center l'objet d'intérêt et à l'orienter de manière identique dans le plan de l'image, des réductions ou des augmentations de taille homothétiques pour la mise à une échelle commune des images à comparer. A l'issue de ces traitements préparatoires, les images résultantes **143** du même objet acquises à des instants différents sont identiques, à l'intérieur d'une tolérance qui est avantageusement déterminée par le système de manière auto adaptative, tant que le grain de beauté ou la lésion n'évolue pas de manière anormale. Il est prévu qu'un problème soit détecté **140** et signalé à un procédé d'ordre supérieur si changement anormal est détecté à la suite de l'exécution de traitements appropriés **144.**
L'ordonnancement dans le temps préalablement à l'application des traitements de comparaison **144** permet de les simplifier en ce que ce qui est recherché pour détecter une anomalie est l'augmentation d'au moins une caractéristique de l'image par rapport à une image acquise antérieurement. En outre, la gestion des instants d'acquisition des images permet avantageusement de calculer des vitesses d'évolution et d'enrichir avec ces informations complémentaires, l'information de signalement de détection d'un problème au procédé d'ordre supérieur. Il est par exemple prévu des actions qui exploitent l'information complémentaire de vitesse d'évolution pour optimiser la gestion du degré d'urgence à consulter un médecin à l'issue de la détection d'un problème.

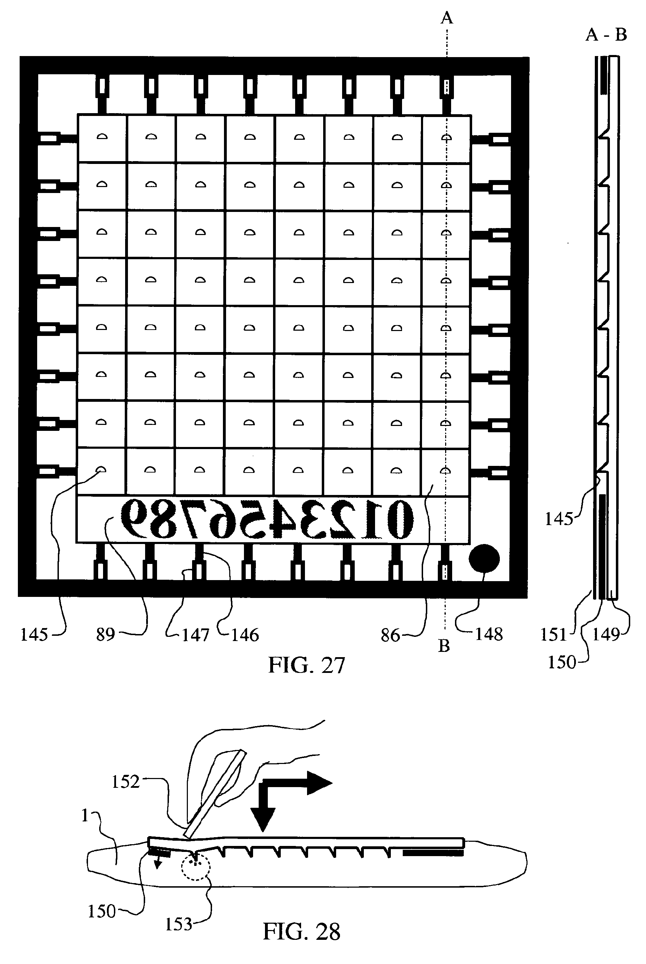

La **figure 27** illustre un exemple de consommable utilisé pour la recherche de substances allergisantes selon l'invention. Le sous ensemble consommable de cet exemple non limitatif est vu de dessous. Le consommable comprend avantageusement le plus grand nombre possible de zones élémentaires **86,** dans une surface prévue pour être utilisée en tant que consommable associé à des moyens pour produire un grossissement optique de l'image selon l'invention tels que décrits dans les exemples précédents. Il est aussi prévu de préférence que les consommables pour la recherche de substances allergisantes soient aptes à une acquisition d'image par un appareil électronique grand public sans qu'il soit nécessaire d'utiliser des moyens pour produire un grossissement optique de l'image. Un accroissement de la surface du consommable est prévu pour pouvoir se dispenser des moyens additionnels de grossissement de l'image. Chacune des zones élémentaires **86** comprend au moins une microlame **145** ou une micropointe sur laquelle est déposée une substance possiblement allergisante. Des moyens sont prévus pour déposer sur la peau des repères permettant aux traitements d'image de localiser l'emplacement ou chaque microlame **145** ou micropointe a provoqué une microlésion de la peau lors de l'application. Avantageusement dans le but d'augmenter la densité de zones élémentaires par consommable, l'ensemble de la grille ne sera pas matérialisé mais seulement des repères périphériques **(146, 147)** le seront. Pour permettre aux traitements d'image de repérer les éléments de localisation quelle que soit la couleur de la peau sur laquelle ils sont appliqués, un repère foncé **146** sera systématiquement associé à un repère clair **147.** Au moins un repère d'orientation globale **148** du consommable sera avantageusement ajouté pour permettre aux traitements d'image de s'adapter au positionnement de l'image du consommable dans l'image telle qu'acquise par l'appareil électronique. Un code d'identification **89** du consommable est nécessaire pour que les substances provoquant une réaction cutanée soient identifiées par les traitements. La coupe **A-B** illustre la structure interne du consommable qui comprend trois couches distinctes dans cet exemple. La couche principale est le support **149** du consommable qui est réalisé dans une matière plastique flexible de faible épaisseur. Le support **149** comprend au centre de chaque zone élémentaire, au moins une microlame **145** ou une micropointe formée par exemple lors du moulage du support. Certaines versions de microlame ou de micropointes comprennent en outre une fonction de microréservoir en leur centre. La matière plastique étant le cas échéant traitée, au moins au niveau des microlames ou des micropointes, pour que les substances déposées y adhérent. Des solutions d'adhérence des substances fondées basées sur la capillarité sont également prévues. La couche **150** est la couche qui assure l'adhésion sur la peau de l'ensemble du consommable au cours des étapes d'application et d'attente du temps de réaction du système immunitaire pour le cas échéant provoquer une réaction cutanée visible. Passé le temps minimum prescrit pour qu'une réaction cutanée apparaisse et avant qu'elle soit susceptible de disparaitre, le support **149** est décollé de la couche **150** au moyen d'un coin non adhérant ou d'une languette à soulever. Le retrait du support **149** laisse alors apparaitre toutes les zones qui ont été exposées aux substances ainsi que les éléments de marquages indispensables aux étapes suivantes. La couche **150** est réalisée dans un film en matière plastique souple ajouré selon les variantes complètement en son centre ou préférentiellement seulement au niveau de chaque zone élémentaire pour réaliser des cloisonnements étanches entre elles évitant des possibilités de migrations et de mélanges de substances entre des zones élémentaires adjacentes. Le film de la couche **150** est recouvert d'adhésifs appropriés sur ses deux faces de sorte que l'adhérence sur la peau soit plus forte que sur la matière plastique du support **149.** Ainsi, le support peut être aisément décollé sans que couche **150** se décolle de la peau. Lors du procédé de fabrication du consommable, les substances à faire pénétrer sous la barrière de protection de la peau sont déposées sur les microlames ou les micropointes après l'assemblage de la couche **150** sur le support **149.** Le film de protection **151** est ensuite posé sur la face de la couche **150** opposée au support **149.** Le film de protection **151** en matière plastique referme de manière étanche à l'air et aux pollutions de toutes sortes l'ensemble du consommable ainsi constitué. Dans les variantes préférées où un cloisonnement est laissé entre les zones élémentaires par des découpes appropriées dans la couche **150,** chaque zone élémentaire est alors refermée par le film de protection formant un mini caisson individuel entièrement étanche. Après l'acquisition d'image, la couche **150** peut alors être décollée de la peau au moyen d'un coin laissé non adhérant ou d'une languette distincts de ceux affectés au décollage du support **149.** Dans des variantes de réalisation à faible coût, la couche **150** peut avantageusement utiliser des solutions de marquage temporaire de la peau par transfert qui sont habituellement utilisées pour fabriquer des tatouages temporaires.
La **figure 28** illustre la pose du consommable utilisé pour la recherche de substance allergisantes sur la surface de peau **1** au moyen d'une petite palette **152,** réalisée en matière plastique rigide, de forme rectangulaire et ayant une largeur correspondant à celle du consommable. La palette est fournie systématiquement avec le consommable. La pose consiste à balayer avec la palette **152** toute la longueur du consommable en appliquant continument une force en direction de la peau. Cette force crée une pression sous la ligne de contact entre la palette et le support **149** qui amènent les microlames **145** ou les micropointes qui sont présentes sous la ligne d'application de la force, à provoquer des microlésions **153** de la peau et à faire pénétrer les substances sous la barrière de protection de la peau. L'application de la force au cours du balayage de la surface du consommable assure en outre une bonne adhésion de la couche **150** sur la peau ou, le cas échéant, un transfert du film dans le cas de l'utilisation de solutions du type tatouage temporaire.

Bien entendu, l'invention n'est pas limitée aux exemples qui viennent d'être décrits et de nombreux aménagements peuvent être apportés à ces exemples, notamment en combinant différemment des éléments pris dans plusieurs exemples, sans sortir du cadre de l'invention.

## Revendications

1. Système pour détecter la sensibilité d'une personne à une ou à plusieurs substances possiblement allergisantes par des moyens relevant de l'optique (4, 3) et du traitement informatique (4, 5) d'image de la peau (1) comprenant :
- Un consommable comprenant l'une ou les plusieurs substances possiblement allergisantes à mettre en contact avec une surface de peau; et **caractérisé en ce qu'**il comprend :
- un appareil électronique grand public (4) comprenant dans un même boitier non spécifique à la détection de la sensibilité d'une personne à une ou plusieurs substances possiblement allergisantes, un objectif, des moyens de mise au point automatique de l'image de la surface de peau d'intérêt à acquérir, des moyens pour acquérir au moins une image par des moyens numériques, des moyens pour produire des données en relation avec l'au moins une image acquise et des moyens pour transmettre des informations par l'intermédiaire d'un réseau; et
- des moyens (4, 5) pour traiter lesdites données en relation avec l'au moins une image acquise dans le but de produire une information en rapport avec la sensibilité d'une personne à l'une ou les plusieurs substances possiblement allergisantes.

2. Système selon la revendication 1, **caractérisé en ce que** le consommable comprend au moins deux parties pouvant être retirées séparément de la surface de peau.

3. Système selon l'une des revendications 1 ou 2, **caractérisé en ce que** le consommable comprend en outre des moyens pour inclure au moins un repère visible dans l'image à acquérir.

4. Système selon l'une quelconque des revendications 1 à 3, **caractérisé en ce que** le consommable comprend en outre des moyens pour occasionner au moins une microlésion de la peau.

5. Système selon l'une quelconque des revendications 1 à 4, **caractérisé en ce que** le consommable comprend une pluralité de zones élémentaires organisées pour pouvoir en déterminer la position dans l'image acquise par traitement d'image et/ou par calcul.

6. Système selon l'une quelconque des revendications 1 à 5, **caractérisé en ce qu'**il comprend en outre des moyens pour produire un grossissement optique de l'image (7).

7. Système selon la revendication 6, **caractérisé en ce que** les moyens pour produire un grossissement optique de l'image comprennent en outre :
- des moyens pour rendre l'image nette lors d'une acquisition d'image à une distance plus courte que la plus courte distance possible pour laquelle ledit appareil électronique grand public a été conçu; et
- des moyens pour fixer temporairement lesdits moyens pour rendre l'image nette sur ledit appareil électronique grand public de sorte que les moyens pour rendre l'image nette soient placés de manière appropriée relativement à l'objectif de l'appareil électronique grand public.

8. Système selon la revendication 6, **caractérisé en ce que** les moyens pour produire un grossissement optique de l'image comprennent en outre :
- des moyens pour rendre l'image nette indépendamment de la distance à laquelle se trouve l'objectif de l'appareil électronique grand public utilisé pour acquérir l'image; et
- des moyens pour maintenir temporairement les moyens pour rendre l'image nette en position fonctionnelle sur la peau.

9. Système selon l'une quelconque des revendications 6 à 8, **caractérisé en ce que** les moyens pour produire un grossissement optique de l'image comprennent en outre des moyens pour déterminer la distance de mise au point par contact entre un élément matériel et une partie du corps de la personne dont on souhaite détecter la sensibilité à une ou plusieurs substances possiblement allergisantes.

10. Système selon l'une quelconque des revendications 1 à 9, **caractérisé en ce que** le consommable et/ou les moyens pour produire un grossissement optique de l'image ont des caractéristiques identifiables par un moyen d'identification inclus dans l'image.

11. Système selon l'une quelconque des revendications 1 à 10, **caractérisé en ce que** le consommable et/ou les moyens pour produire un grossissement optique de l'image comprennent en outre des moyens pour permettre à l'utilisateur d'entrer au moins un élément d'information dans l'image.

12. Système selon l'une quelconque des revendications 1 à 11, **caractérisé en ce que** le consommable et/ou les moyens pour produire un grossissement optique de l'image comprennent en outre des moyens pour étalonner au moins une caractéristique de l'image.

13. Système selon l'une quelconque des revendications 1 à 12, **caractérisé en ce que** le consommable et/ou les moyens pour produire un grossissement optique de l'image comprennent en outre des moyens pour apporter au moins une information à l'utilisateur.

14. Système selon l'une quelconque des revendications 1 à 13, **caractérisé en ce que** le consommable et/ou les moyens pour produire un grossissement optique de l'image sont agencés pour être livrés aux utilisateurs sous une forme de faible épaisseur utilisant des matériaux flexibles.

15. Procédé pour détecter la sensibilité d'une personne à une ou plusieurs substances possiblement allergisantes par des moyens relevant de l'optique et du traitement informatique d'image de la peau, **caractérisé en ce qu'**il comprend des étapes au cours desquelles :
- On met en contact une surface de peau avec l'une ou les plusieurs substances possiblement allergisantes par l'intermédiaire d'un consommable; et
- On acquière au moins une image numérique comprenant une surface de peau ayant été mise en contact avec l'une ou les plusieurs substances possiblement allergisantes au moyen d'un appareil électronique grand public comprenant dans un même boitier non spécifique à la détection de la sensibilité d'une personne à une ou plusieurs substances possiblement allergisantes, un objectif, des moyens de mise au point automatique de l'image de la surface de peau d'intérêt à acquérir, des moyens pour acquérir au moins une image par des moyens numériques, des moyens pour produire des données en relation avec l'au moins une image acquise et des moyens pour transmettre des informations par l'intermédiaire d'un réseau; et
- On récupère des données en relation avec l'au moins une image acquise dans un format apte aux traitements d'image; et
- On traite les données récupérées qui sont en relation avec l'image acquise pour produire au moins une information en rapport avec la sensibilité d'une personne à l'une ou les plusieurs substances possiblement allergisantes.

16. Procédé pour détecter la sensibilité d'une personne à une ou plusieurs substances possiblement allergisantes selon la revendication 15, **caractérisé en ce qu'**il comprend en outre une étape au cours de laquelle :
- On place des moyens pour produire un grossissement optique de l'image préalablement à son acquisition entre l'objectif dudit appareil électronique grand public et la surface de peau.

17. Procédé pour détecter la sensibilité d'une personne à une ou plusieurs substances possiblement allergisantes selon l'une quelconque des revendications 15 ou 16, **caractérisé en ce qu'**il comprend en outre une étape au cours de laquelle :
- On retire, préalablement à l'acquisition de l'au moins une image, au moins une partie du consommable après le temps minimum nécessaire pour qu'une réaction cutanée apparaisse et avant qu'elle soit susceptible de disparaitre.

18. Procédé pour détecter la sensibilité d'une personne à une ou plusieurs substances possiblement allergisantes selon l'une quelconque des revendications 15 à 17, **caractérisé en ce qu'**il comprend en outre une étape au cours de laquelle :
- On corrige, l'au moins une image de la peau telle qu'acquise au moyen d'un appareil électronique grand public, et/ou les données issues du traitement de l'au moins une image, en fonction d'informations d'étalonnage qui sont introduites dans l'image par le consommable et/ou par les moyens pour produire un grossissement optique de l'image.

19. Procédé pour détecter la sensibilité d'une personne à une ou plusieurs substances possiblement allergisantes selon l'une quelconque des revendications 15 à 18, **caractérisé en ce qu'**il comprend en outre une étape au cours de laquelle :
- On détecte la présence ou l'absence d'une réaction cutanée visible par un changement de couleur et/ou par un changement de relief cutané localisé à l'endroit de la mise en contact avec la substance possiblement allergisante.

20. Procédé pour détecter la sensibilité d'une personne à une ou plusieurs substances possiblement allergisantes selon l'une quelconque des revendications 15 à 19, **caractérisé en ce qu'**il comprend en outre une étape au cours de laquelle :
- On rapproche les informations de localisation d'une réaction cutanée visible dans une image, la connaissance de la localisation correspondante sur le consommable de la substance ayant provoqué la réaction cutanée et la connaissance de la nature de la substance qui était déposée à l'endroit correspondant sur le consommable pour produire au moins une information en rapport avec la nature de la substance ayant provoqué la réaction cutanée.

21. Procédé pour détecter la sensibilité d'une personne à une ou plusieurs substances possiblement allergisantes selon l'une quelconque des revendications 15 à 20, **caractérisé en ce qu'**il comprend en outre une étape au cours de laquelle :
- On transmet au moins une information à un serveur distant à partir d'un appareil grand public connecté à un réseau.

22. Procédé pour détecter la sensibilité d'une personne à une ou plusieurs substances possiblement allergisantes selon l'une quelconque des revendications 15 à 21, **caractérisé en ce qu'**il comprend en outre une étape au cours de laquelle :
- On remet à l'utilisateur au moins une partie des résultats attendus par la réception d'au moins une information venant d'au moins un serveur distant.

## Patentansprüche

1. System zur Erkennung der Sensibilität einer Person gegenüber einem oder mehreren möglicherweise allergieauslösenden Stoffen durch optische Mittel (4, 3) und digitale Bildverarbeitung (4, 5) des Bildes der Haut (1), die Folgendes umfassen:
- Ein Verbrauchsmittel, zu dem ein oder mehrere möglicherweise allergieauslösende Stoffe gehören, die mit einer Hautstelle in Kontakt gebracht werden sollen; und das **dadurch gekennzeichnet ist, dass** es Folgendes umfasst:
- Ein elektronisches Verbrauchergerät (4), zu dem in ein und demselben Gehäuse, das für die Erkennung der Sensibilität einer Person gegenüber einem oder mehreren möglicherweise allergieauslösenden Stoffen nicht spezifisch ist, ein Objektiv gehört, Mittel für die automatische Fokuseinstellung des Bildes der betroffenen Hautstelle, die erfasst werden soll, Mittel, mit denen mindestens ein Bild durch digitale Mittel erfasst werden kann, Mittel, um Daten in Bezug auf mindestens ein erfasstes Bild zu erzeugen und Mittel, um Informationen mittels eines Netzwerks zu übermitteln; und
- Mittel (4, 5) zur Verarbeitung der genannten Daten hinsichtlich mindestens eines der erfassten Bilder mit dem Ziel, eine Information im Zusammenhang mit der Sensibilität einer Person gegenüber einem oder mehreren möglicherweise allergieauslösenden Stoffen zu generieren.

2. System gemäß Anspruch 1, das **dadurch gekennzeichnet ist, dass** das Verbrauchsmittel aus mindestens zwei Teilen besteht, die getrennt voneinander von der Hautstelle entfernt werden können.

3. System nach einem der Ansprüche 1 oder 2, das **dadurch gekennzeichnet ist, dass** das Verbrauchsmittel des Weiteren über Mittel verfügt, um mindestens eine sichtbare Markierung in das zu erfassende Bild einzuschließen.

4. System nach einem der Ansprüche 1 bis 3, das **dadurch gekennzeichnet ist, dass** das Verbrauchsmittel des Weiteren über Mittel verfügt, um mindestens eine Mikroläsion der Haut zu verursachen.

5. System nach einem der Ansprüche 1 bis 4, das **dadurch gekennzeichnet ist, dass** zum Verbrauchsmittel mehrere elementare Bereiche gehören, die so angeordnet sind, dass die diesbezügliche Position im erfassten Bild mittels Bildverarbeitung bzw. Berechnung bestimmt werden kann.

6. System nach einem der Ansprüche 1 bis 5, das **dadurch gekennzeichnet ist, dass** es des Weiteren über Mittel zur Erzeugung einer optischen Vergrößerung des Bildes verfügt (7).

7. System nach Anspruch 6, das **dadurch gekennzeichnet ist, dass** die genannten Mittel zur Erzeugung einer optischen Vergrößerung des Bildes des Weiteren über Folgendes verfügen:
- Mittel, um das Bild bei einer Bilderfassung bei einem kleineren Abstand als dem kleinstmöglichen Abstand, für den das genannte elektronische Verbrauchergerät entwickelt wurde, schärfer darzustellen; und
- Mittel, um vorübergehend die genannten Mittel, um das Bild schärfer darzustellen, am genannten elektronischen Verbrauchergerät zu befestigen, so dass die Mittel, um das Bild schärfer darzustellen, in geeigneter Weise zum Objektiv des elektronischen Verbrauchergeräts positioniert werden.

8. System nach Anspruch 6, das **dadurch gekennzeichnet ist, dass** die genannten Mittel zur Erzeugung einer optischen Vergrößerung des Bildes des Weiteren über Folgendes verfügen:
- Mittel, um das Bild unabhängig vom Abstand, in dem sich das Objektiv zum elektronischen Verbrauchergerät befindet, das zum Erfassen des Bildes verwendet wurde, scharf darzustellen; und
- Mittel, um vorübergehend die Mittel, um das Bild scharf darzustellen, in Betriebsposition auf der Haut zu halten.

9. System nach einem der Ansprüche 6 bis 8, das **dadurch gekennzeichnet ist, dass** die Mittel zur Erzeugung einer optischen Vergrößerung des Bildes des Weiteren über Mittel verfügen, um die Fokusentfernung festzulegen durch Kontakt zwischen einem materiellen Element und einem Teil des Körpers der Person, deren Sensibilität gegenüber einem oder mehreren möglicherweise allergieauslösenden Stoffen erfasst werden soll.

10. System nach einem der Ansprüche 1 oder 9, das **dadurch gekennzeichnet ist, dass** das Verbrauchsmittel bzw. die Mittel zur Erzeugung einer optischen Vergrößerung des Bildes über Merkmale verfügen, die anhand eines im Bild enthaltenen Erkennungsmerkmals identifiziert werden können.

11. System nach einem der Ansprüche 1 bis 10, das **dadurch gekennzeichnet ist, dass** das Verbrauchsmittel bzw. die Mittel zur Erzeugung einer optischen Vergrößerung des Bildes des Weiteren über Mittel verfügen, anhand derer der Anwender mindestens eine Information in das Bild eingeben kann.

12. System nach einem der Ansprüche 1 bis 11, das **dadurch gekennzeichnet ist, dass** das Verbrauchsmittel bzw. die Mittel zur Erzeugung einer optischen Vergrößerung des Bildes des Weiteren über Mittel verfügen, anhand derer der Anwender mindestens ein Merkmal des Bildes kalibrieren kann.

13. System nach einem der Ansprüche 1 bis 12, das **dadurch gekennzeichnet ist, dass** das Verbrauchsmittel bzw. die Mittel zur Erzeugung einer optischen Vergrößerung des Bildes des Weiteren über Mittel verfügen, um dem Anwender mindestens eine Information zu erbringen.

14. System nach einem der Ansprüche 1 bis 13, das **dadurch gekennzeichnet ist, dass** das Verbrauchsmittel bzw. die Mittel zur Erzeugung einer optischen Vergrößerung des Bildes so angeordnet sind, dass sie den Anwendern in geringer Dicke geliefert werden können, wobei flexible Materialien verwendet werden.

15. Verfahren zur Erkennung der Sensibilität einer Person gegenüber einem oder mehreren möglicherweise allergieauslösenden Stoffen durch optische Mittel und digitale Bildverarbeitung der Haut, das **dadurch gekennzeichnet ist, dass** es die folgenden Schritte umfasst, in denen:
- Eine Hautstelle mit einem oder mehreren möglicherweise allergieauslösenden Stoffen mittels eines Verbrauchsmittels in Kontakt gebracht werden; und
- Man mindestens ein digitales Bild erfasst, das einer Hautstelle entspricht, die mit einem oder mehreren möglicherweise allergieauslösenden Stoffen in Kontakt gebracht wurde mittels eines elektronischen Verbrauchergeräts, zu dem in ein und demselben Gehäuse, das für die Erkennung der Sensibilität einer Person gegenüber einem oder mehreren möglicherweise allergieauslösenden Stoffen nicht spezifisch ist, ein Objektiv gehört, Mittel für die automatische Fokuseinstellung des Bildes der betroffenen Hautstelle, die erfasst werden soll, Mittel, mit denen mindestens ein Bild durch digitale Mittel erfasst werden kann, Mittel, um Daten in Bezug auf mindestens ein erfasstes Bild zu erzeugen und Mittel, um Informationen mittels eines Netzwerks zu übermitteln; und
- Daten in Bezug auf mindestens ein erfasstes Bild in einem Format abgerufen werden, das für die Bildverarbeitung geeignet ist; und
- Die abgerufenen Daten in Bezug auf das erfasste Bild so bearbeitet werden, dass mindestens eine Information im Zusammenhang mit der Sensibilität einer Person gegenüber einem oder mehreren möglicherweise allergieauslösenden Stoffen generiert wird.

16. Verfahren zur Erfassung der Sensibilität einer Person gegenüber einem oder mehreren möglicherweise allergieauslösenden Stoffen nach Anspruch 15, das **dadurch gekennzeichnet ist, dass** es des Weiteren den folgenden Schritt umfasst, in dem:
- Mittel, um eine optische Vergrößerung des Bildes vor seiner Erfassung zu erzeugen, zwischen dem Objektiv des genannten elektronischen Verbrauchergeräts und der Hautstelle positioniert werden.

17. Verfahren zur Erfassung der Sensibilität einer Person gegenüber einem oder mehreren möglicherweise allergieauslösenden Stoffen nach einem der Ansprüche 15 oder 16, das **dadurch gekennzeichnet ist, dass** es des Weiteren den folgenden Schritt umfasst, in dem:
- Man vor der Erfassung mindestens eines Bildes mindestens einen Teil des Verbrauchsmittels nach der minimalen Zeit entfernt, die erforderlich ist, damit eine Hautreaktion auftritt und bevor sie wieder verschwinden kann.

18. Verfahren zur Erfassung der Sensibilität einer Person gegenüber einem oder mehreren möglicherweise allergieauslösenden Stoffen nach einem der Ansprüche 15 bis 17, das **dadurch gekennzeichnet ist, dass** es des Weiteren den folgenden Schritt umfasst, in dem:
- Mindestens ein Bild der Haut, wie es mittels eines elektronischen Verbrauchergeräts erfasst wurde, bzw. Daten aus der Verarbeitung mindestens eines Bildes, in Bezug auf die Kalibrierungsinformationen, die in das Bild durch das Verbrauchsmittel bzw. die Mittel zur Erzeugung einer optischen Vergrößerung des Bildes eingefügt werden, korrigiert werden.

19. Verfahren zur Erfassung der Sensibilität einer Person gegenüber einem oder mehreren möglicherweise allergieauslösenden Stoffen nach einem der Ansprüche 15 bis 18, das **dadurch gekennzeichnet ist, dass** es des Weiteren den folgenden Schritt umfasst, in dem:
- Man das Auftreten oder Fehlen einer Hautreaktion erfasst, die durch eine Veränderung der Farbe bzw. durch eine Veränderung des Reliefs der Haut an der Stelle sichtbar wird, wo die Haut mit dem möglicherweise allergieauslösenden Stoff in Kontakt gebracht wurde.

20. Verfahren zur Erfassung der Sensibilität einer Person gegenüber einem oder mehreren möglicherweise allergieauslösenden Stoffen nach einem der Ansprüche 15 bis 19, das **dadurch gekennzeichnet ist, dass** es des Weiteren den folgenden Schritt umfasst, in dem:
- Man die Informationen bezüglich der Lokalisierung einer in einem Bild sichtbaren Hautreaktion, die Kenntnis der entsprechenden Lokalisierung auf dem Verbrauchsmittel des Stoffes, der die Hautreaktion ausgelöst hat, und die Kenntnis der Art der Substanz, die an der entsprechenden Stelle auf dem Verbrauchsmittel aufgetragen wurde, miteinander in Beziehung setzt, um mindestens eine Information hinsichtlich der Art der Substanz zu erzeugen, die die Hautreaktion ausgelöst hat.

21. Verfahren zur Erfassung der Sensibilität einer Person gegenüber einem oder mehreren möglicherweise allergieauslösenden Stoffen nach einem der Ansprüche 15 bis 20, das **dadurch gekennzeichnet ist, dass** es des Weiteren den folgenden Schritt umfasst, in dem:
- Mindestens eine Information an einen Remoteserver mittels eines Verbrauchergeräts, das an ein Netz angeschlossen ist, übermittelt wird.

22. Verfahren zur Erfassung der Sensibilität einer Person gegenüber einem oder mehreren möglicherweise allergieauslösenden Stoffen nach einem der Ansprüche 15 bis 21, das **dadurch gekennzeichnet ist, dass** es des Weiteren den folgenden Schritt umfasst, in dem:
- Dem Anwender mindestens ein Teil der erwarteten Ergebnisse durch den Empfang mindestens einer Information, die mindestens von einem Remoteserver stammt, vorgelegt wird.

## Claims

1. A system for detecting the sensitivity of a person to one or more potentially allergenic substances by means falling under optics (4, 3) and data processing (4, 5) of image of the skin (1) comprising:
- A consumable comprising one or more potentially allergenic to be put in contact with a skin surface; and **characterized in that** it comprises:
- a consumer electronics device (4) comprising in a common housing which is not specific to detection of the sensitivity of a person to one or more potentially allergenic substances, means for automatic focusing of the image of the skin surface of interest to be acquired, means for acquiring at least one image by digital means, means for producing data related to the at least one acquired image and means for transmitting information via a network; and
- means (4, 5) for processing said data related to the at least one acquired image in order to produce information related to the sensitivity of a person to one or more potentially allergenic substances.

2. System according to claim 1, **characterized in that** the consumable comprises at least two parts which can be removed separately from the skin surface.

3. System according to one of Claims 1 or 2, **characterized in that** the consumable further comprises means for including at least one visible mark in the image to be acquired.

4. System according to any one of claims 1 to 3, **characterized in that** the consumable further comprises means for causing at least one microlesion on the skin.

5. System according to any one of claims 1 to 4, **characterized in that** the consumable comprises a plurality of elementary areas organized in order to be able to determine the position thereof in the acquired image by image processing and/or by calculation.

6. System according to any one of claims 1 to 5, **characterized in that** it further comprises means for producing an optical magnification of the image (7).

7. System according to claim 6, **characterized in that** the means for producing an optical magnification of the image further comprise:
- Means for sharpening the image during an image acquisition at a shorter distance than the shortest distance possible for which said consumer electronics device has been designed; and
- means for temporarily attaching said means for sharpening the image on said consumer electronics device so that the means for sharpening the image are suitably placed relatively to the objective of the consumer electronics device.

8. System according to claim 6, **characterized in that** the means for producing an optical magnification of the image further comprise:
- Means for sharpening the image independently of the distance at which is found the objective of the consumer electronics device used for acquiring the image; and
- means for temporarily maintaining the means for sharpening the image in a functional position on the skin.

9. System according to any one of claims 6 to 8, **characterized in that** the means for producing an optical magnification of the image further comprise means for determining the focusing distance by contact between a hardware element and a portion of the body of the person, of whom the detection of the sensitivity to one or more possibly allergenic substances is wished.

10. System according to any one of claims 1 to 9, **characterized in that** the consumable and/or the means for producing an optical magnification of the image have characteristics which may be identified by identification means included in the image.

11. System according to any one of claims 1 to 10, **characterized in that** the consumable and/or the means for producing an optical magnification of the image further comprise means for allowing the user to enter at least one information element in the image.

12. System according to any one of claims 1 to 11, **characterized in that** the consumable and/or the means for producing an optical magnification of the image further comprise means for calibrating at least one characteristic of the image.

13. System according to any one of claims 1 to 12, **characterized in that** the consumable and/or the means for producing an optical magnification of the image further comprise means for providing at least one information to the user.

14. System according to any one of claims 1 to 13, **characterized in that** the consumable and/or the means for producing optical magnification of the image are laid out so as to be delivered to the users in a form having small thickness using flexible materials.

15. A method for detecting the sensitivity of a person to one or more potentially allergenic substances by means falling under optics and data processing of image of the skin, **characterized in that** it comprises steps during which:
- A skin surface is put into contact with one or more possibly allergenic substances via a consumable; and
- at least one digital image is acquired, comprising a skin surface having been put into contact with one or more possibly allergenic substances, by means of a consumer electronics device comprising in a common housing that is not specific to detection of the sensitivity of a person to one or more potentially allergenic substances, means for automatic focusing of the image of the skin surface of interest to be acquired, means for acquiring at least one image by digital means, means for producing data related to the at least one acquired image and means for transmitting information via a network; and
- data related to said at least one acquired image are recovered in a format suitable for image processing operations; and
- the recovered data which are in connection with the acquired image for producing at least one information related to the sensitivity of a person to one or more possibly allergenic substances, are processed.

16. Method for detecting the sensitivity of a person to one or more potentially allergenic substances according to claim 15, **characterized in that** it further comprises a step during which:
- Are placed means for producing optical magnification of the image before its acquisition between the objective of said consumer electronics device and the skin surface.

17. Method for detecting the sensitivity of a person to one or more potentially allergenic substances according to any one of claims 15 or 16, **characterized in that** it further comprises a step during which:
- Is withdrawn, before the acquisition of said at least one image, at least one portion of the consumable after the minimum time required for a skin reaction to appear and before it is likely to disappear.

18. Method for detecting the sensitivity of a person to one or more potentially allergenic substances according to any one of claims 15 to 17, **characterized in that** it further comprises a step during which:
- Is corrected, the at least one image of the skin as acquired by a consumer electronics device, and/or the processed data of the at least one image, based on information of calibration which are introduced into the image by the consumable and/or by the means for producing an optical magnification of the image.

19. Method for detecting the sensitivity of a person to one or more potentially allergenic substances according to any one of claims 15 to 18, **characterized in that** it further comprises a step during which:
- The presence or the absence of a visible skin reaction is detected by a change of color and/or by a change of skin relief localized at the location of contact with the possibly allergenic substance.

20. Method for detecting the sensitivity of a person to one or more potentially allergenic substances according to any one of claims 15 to 19, **characterized in that** it further comprises a step during which:
- Information related to the localization information of a skin reaction visible in an image, the knowledge of the corresponding localization on the consumable of the substance having caused the skin reaction, and the knowledge of the nature of the substance which was deposited at the corresponding location on the consumable, are associated in order to produce at least one information related to the nature of the substance having caused the skin reaction.

21. Method for detecting the sensitivity of a person to one or more potentially allergenic substances according to any one of claims 15 to 20, **characterized in that** it further comprises a step during which:
- At least information is sent to a remote server from a consumer electronics device connected to a network.

22. Method for detecting the sensitivity of a person to one or more potentially allergenic substances according to any one of claims 15 to 21, **characterized in that** it further comprises a step during which:
- At least a part of expected results is presented to the user by the receiving of at least one information from at least one remote server.
